# EUROPEAN PATENT SPECIFICATION

(11) **EP 1 370 693 B1**
(45) Date of publication and mention of the grant of the patent: **04.06.2008**
(21) Application number: 02723130.7
(22) Date of filing: 12.02.2002
(51) Int. Cl.: C12Q 1/68

(54) **AMPLIFIED CANCER GENE WIP1**
VERSTÄRKTES KREBS-GEN WIP1
GENE DU CANCER AMPLIFIE WIP1

(30) Priority: 14.02.2001 US 268362 P
(43) Date of publication of application: 17.12.2003
(73) Proprietor: Amgen Inc., Thousand Oaks, CA 91320-1799 (US)
(72) Inventor: LI, Jing, Apt. 5C, Flushing NY 11355 (US); POWERS, Scott, Greenlawn, NY 11740 (US)
(74) Representative: Wakerley, Helen Rachael
(86) International application number: PCT/US2002/003991
(87) International publication number: WO 2002/064838

(56) References cited:
- WO-A-99/44063
- FISCELLA M ET AL: "Wip1, a novel human rotein phosphatase that is induced in response to ionizing radiation in a p53-dependent manner" PROCEEDINGS OF THE NATIONAL ACADEMY OF SCIENCES OF USA, NATIONAL ACADEMY OF SCIENCE. WASHINGTON, US, vol. 94, June 1997 (1997-06), pages 6048-6053, XP002132303 ISSN: 0027-8424 cited in the application
- TAKEKAWA MUTSUHIRO ET AL: "p53-inducible Wip1 phosphatase mediates a negative feedback regulation of p38 MAPK-p53 signaling in response to UV radiation." EMBO (EUROPEAN MOLECULAR BIOLOGY ORGANIZATION) JOURNAL, vol. 19, no. 23, 1 December 2000 (2000-12-01), pages 6517-6526, XP002232935 ISSN: 0261-4189 cited in the application
- KNUUTILA S ET AL: "DNA COPY NUMBER AMPLIFICATIONS IN HUMAN NEOPLASMS REVIEW OF COMPARATIVE GENOMIC HYBRIDIZATION STUDIES" AMERICAN JOURNAL OF PATHOLOGY, PHILADELPHIA, PA, US, vol. 152, no. 5, May 1988 (1988-05), pages 1107-1123, XP001004427 ISSN: 0002-9440 cited in the application
- BAERLUND M ET AL: "MULTIPLE GENES AT 17Q23 UNDERGO AMPLIFCATION AND OVEREXPRESSION IN BREAST CANCER" CANCER RESEARCH, AMERICAN ASSOCIATION FOR CANCER RESEARCH, BALTIMORE, MD, US, vol. 60, no. 19, 1 October 2000 (2000-10-01), pages 5340-5344, XP001096150 ISSN: 0008-5472
- POLLACK J R ET AL: "Genome-wide analysis of DNA copy-number changes using cDNA microarrays" NATURE GENETICS, NATURE AMERICA, NEW YORK, US, vol. 23, no. 1, September 1999 (1999-09), pages 41-46, XP002221379 ISSN: 1061-4036 cited in the application

## Description

This application relates to U. S. Serial No. 60/268,362, filed February 14, 2001.

### BACKGROUND OF THE INVENTION

### 1. Field of the Invention

The present invention relates to oncogenes and to cancer diagnostics and therapeutics. More specifically, the present invention relates an amplified and overexpressed WIP1 gene is involved in certain types of cancers. The invention pertains to the amplified gene, its encoded proteins, and antibodies, inhibitors, activators and the like in cancer screening and anti-cancer therapy, including breast cancer.

### 2. Background of the Invention

Cancer is the second leading cause of death in the United States, after heart disease (Boring, el al., CA Cancer J. Clin., 43:7, 1993), and it develops in one in three Americans. One of every four Americans dies of cancer. Cancer features uncontrolled cellular growth, which results either in local invasion of normal tissue or systemic spread of the abnormal growth known as metastasis. A particular type of cancer or a particular stage of cancer development may involve both elements.

The division or growth of cells in various tissues functioning in a living body normally takes place in an orderly and controlled manner. This is enabled by a delicate growth control mechanism, which involves, among other things, contact, signaling, and other communication between neighboring cells. Growth signals, stimulatory or inhibitory, are routinely exchanged between cells in a functioning tissue. Cells normally do not divide in the absence of stimulatory signals, and will cease dividing when dominated by inhibitory signals. However, such signaling or communication becomes defective or completely breaks down in cancer cells. As a result, the cells continue to divide; they invade adjacent structures, break away from the original tumor mass, and establish new growth in other parts of the body. The latter progression to malignancy is referred to as "metastasis."

Cancer generally refers to malignant tumors, rather than benign tumors. Benign tumor cells are similar to normal, surrounding cells. These types of tumors are almost always encapsulated in a fibrous capsule and do not have the potential to metastasize to other parts of the body. These tumors affect local organs but do not destroy them; they usually remain small without producing symptoms for many years. Treatment becomes necessary only when the tumors grow large enough to interfere with other organs. Malignant tumors, by contrast, grow faster than benign tumors; they penetrate and destroy local tissues. Some malignant tumors may spread throughout the body via blood or the lymphatic system. The unpredictable and uncontrolled growth makes malignant cancers dangerous, and fatal in many cases. These tumors are not morphologically typical of the original tissue and are not encapsulated. Malignant tumors commonly recur after surgical removal.

Treatment, therefore, ordinarily targets malignant cancers or malignant tumors. The intervention of malignant growth is most effective at the early stage of the cancer development. It is thus exceedingly important to discover sensitive markers for early signs of cancer formation and to identify potent growth suppression agents associated therewith. The invention of such diagnostic and treatment agents hinges upon the understanding of the genetic control mechanisms for cell division and differentiation, particularly in connection to tumorigenesis. Cancer is caused by inherited or acquired mutations in cancer genes, which have normal cellular functions and which induce or otherwise contribute to cancer once mutated or expressed at an abnormal level. Certain well-studied tumors carry several different independently mutated genes, including activated oncogenes and inactivated tumor suppressor genes. Each of these mutations appears to be responsible for imparting some of the traits that, in aggregate, represent the full neoplastic phenotype (Land et al., Science, 222:771, 1983; Ruley, Nature, 4:602, 1983; Hunter, Cell, 64:249,1991).

One such mutation is gene amplification. Gene amplification involves a chromosomal region bearing specific genes undergoing a relative increase in DNA copy number, thereby increasing the copies of any genes that are present. In general, gene amplification results in increased levels of transcription and translation, producing higher amounts of the corresponding gene mRNA and protein. Amplification of genes causes deleterious effects, which contribute to cancer formation and proliferation (Lengauer et al. Nature, 396:643-649 (1999)).

It is commonly appreciated by cancer researchers that whole collections of genes are demonstrably overexpressed or differentially expressed in a variety of different types of tumor cells. Yet, only a very small number of these overexpressed genes are likely to be causally involved in the cancer phenotype. The remaining overexpressed genes likely are secondary consequences of more basic primary events, for example, overexpression of a cluster of genes, involved in DNA replication. On the other hand, gene amplification is established as an important genetic alteration in solid tumors (Knuutila et al., Am J Pathol 1998 152(5):1107-23; Knuutila *et al., Cancer Genet Cytogenet.* 0:2- (1998)).

The overexpression of certain well known genes, for example, *c-myc,* have been observed at fairly high levels in the absence of gene amplification (Yoshimoto et al., 1986, JPN J Cancer Res, 77(6):540-5), although these genes are frequently amplified (Knuutila et al., Am J Pathol 1998 152(5):1107-23) and thereby activated. Such a characteristic is considered a hallmark of oncogenes. Overexpression in the absence of amplification may be caused by higher transcription efficiency in those situations. In the case of *c-myc,* for example, Yoshimoto *et al.* showed that its transcriptional rate was greatly increased in the tested tumor cell lines. The characteristics and interplay of overexpression and amplification of a gene in cancer tissues, therefore, provide significant indications of the gene's role in cancer development. That is, increased DNA copies of certain genes in tumors, along with and beyond its overexpression, may point to their functions in tumor formation and progression.

Thus, the invention, as well characterization of amplified cancer genes, in general, along with and in addition to their features of overexpression or differential expression, will be a promising avenue that leads to novel targets for diagnostic and therapeutic applications in cancer.

Additionally, the completion of the working drafts of the human genome and the paralleled advances in genomics technologies offer new promises in the identification of effective cancer markers and the anti-cancer agents. The high-throughput microarray detection and screening technology, computer-empowered genetics and genomics analysis tools, and multi-platform functional genomics and proteomics validation systems, all lend themselves in applications in cancer research and findings.

With the advent of modem sequencing technologies and genomic analyses, many unknown genes and genes with unknown or partially known functions are revealed.

It is apparent, therefore, that identification of amplified and/or overexpressed genes, including oncogenes, that are involved in tumorigenesis and cancer progression are desired. It is also apparent that methods of using these genes in cancer diagnosis and treatment are highly desirable. The technologies and knowledge thus call for the invention of novel targets for the diagnostic markers involved in tumorigenesis and new potent anticancer treatment regimen.

### SUMMARY OF THE INVENTION

The present invention relates to isolation, characterization, overexpression and implication of genes, including amplified genes, in cancers, methods and compositions for the diagnosis, prevention, and treatment of tumors and cancers, for example, breast cancer, lung cancer, prostate cancer, ovarian cancer, or colon cancer, *etc.,* in mammals, for example, humans. The invention is based on the finding of novel traits of a protein phosphatase gene, WIP1, which is originally identified as a gene that is induced under UV or gamma radiation under the regulation of p53.

WIP1 gene encodes protein phosphatase, which is expressed in human tumors. As disclosed herein, WIP1 gene appears to be at the epicenter of amplification region in quantitative PCR analysis of human malignant tumors, for example, breast cancer. As disclosed for the first time, WIP1 gene is amplified and overexpressed in over 15% of human breast tumor samples.

These novel traits include the overexpression of the WIP1 gene in certain cancers, for example, breast cancer, lung cancer, prostate cancer, ovarian cancer, or colon cancer, *etc.,* and the frequent amplification of WIP 1 DNA in cancer cells. The WIP1 gene and its expressed protein product can thus be used diagnostically or as targets for cancer therapy; and they can also be used to identify and design compounds useful in the diagnosis, prevention, and therapy of tumors and cancers (for example, breast cancer, lung cancer, prostate cancer, ovarian cancer, colon cancer, *etc*.).

According to one aspect of the present invention, the use of WIP1 in gene therapy, development of antisense nucleic acids and small interfering RNAs (siRNAs), and development of immunodiagnostics or immunotherapy are provided. The present invention also includes production and the use of antibodies, for example, monoclonal, polyclonal, single-chain and engineered antibodies (including humanized antibodies) and fragments, which specifically bind WIP1 proteins and polypeptides. The invention also features antagonists and inhibitors of WIP1 proteins that can inhibit one or more of the functions or activities of WIP1 proteins. Suitable antagonists can include small molecules (molecular weight below about 500), large molecules (molecular weight above about 500), antibodies, including fragments and single chain antibodies, that bind and "neutralize" WIP1 proteins, polypeptides and which compete with a native form of WIP1 proteins for binding to a protein which may naturally interact with WIP1 proteins for the latter's function, and nucleic acid molecules that interfere with transcription of the WIP1 genes (for example, antisense nucleic acid molecules, ribozymes and small interfering RNAs (siRNAs). Useful agonists, ones that may induce certain mutants of WIP1 thereby attenuating activities of WIP1, also include small and large molecules, and antibodies other than "neutralizing" antibodies.

The present invention further features molecules that can decrease the expression of WIP 1 by affecting transcription or translation. Small molecules (molecular weight below about 500), large molecules (molecular weight above about 500), and nucleic acid molecules, for example, ribozymes, siRNAs and antisense molecules may all be utilized to inhibit the expression or amplification.

As mentioned above, the WIP 1 gene sequence also can be employed in an RNA interference context. The phenomenon of RNA interference is described and discussed in Bass, Nature 411 : 428-29 (2001); Elbahir et al., Nature 411: 494-98(2001) ; and Fire et al., Nature 391: 806-11 (1998), where methods of making interfering RNA also are discussed.

In one aspect, the present invention provides a method for diagnosing a cancer, for example, a breast cancer, a lung cancer, a prostate cancer, an ovarian cancer, or a colon cancer, *etc.,* in a mammal, which comprises determining the WIP1 gene copy number in a test sample from a region of the mammal that is suspected to be precancerous or cancerous, thereby generating data for a test gene copy number; and comparing the test gene copy number to data for a control gene copy number, wherein an amplification of the gene indicates the presence of a precancerous lesion or a cancer in the mammal.

In another aspect, the present invention provides a method for diagnosing a cancer, for example, a breast cancer, a lung cancer, a prostate cancer, an ovarian cancer, or colon cancer in a mammal, which comprises determining the level of WIP1 protein expression or WIP1 mRNA transcripts in a test sample from a region of the mammal that is suspected to be precancerous or cancerous thereby generating data for a test level; and comparing the test level to data for a control level, wherein an elevated test level relative to the control level indicates the presence of a precancerous lesion or cancer in the mammal. The control level may be obtained from a region of the mammal that is normal. Alternatively, the control level may be obtained from a different individual or be a normalized value based on baseline values found in a population.

The data may be stored in electronic or paper format. Electronic format can be selected from the group consisting of electronic mail, disk, compact disk (CD), digital versatile disk (DVD), memory card, memory chip, ROM or RAM, magnetic optical disk, tape, video, video clip, microfilm, internet, shared network, shared server and the like; wherein data is displayed, transmitted or analyzed via physical transfer, electronic transmission, video display or telecommunication; wherein data is compared and compiled at the site of sampling specimens or at a location where the data is transported following a process as described above.

In another aspect, the present invention provides use in the manufacture of a medicament for preventing, controlling, or suppressing cancer growth or precancerous growth in a mammalian tissue, for example, in the breast, lung, colon, ovary, or prostate, of a nucleotide molecule which interacts with WIP1 DNA or RNA to inhibit WIP1 gene function. Such nucleotide molecule may be an siRNA, an antisense oligonucleotide, or a ribozyme of WIP1 RNA.

Small interfering RNA (siRNA) may be used, wherein the siRNA comprises less than 100 bps in length.

In another aspect, the present invention provides use in the manufacture of a medicament for preventing, controlling, or suppressing cancer growth or precancerous growth in a mammalian tissue, for example, in the breast, lung, colon, ovary, or prostate, of an antibody to WIP1 protein.

The present invention further provides use in the manufacture of a medicament for preventing, controlling, or suppressing cancer growth or precancerous growth in a mammalian tissue, for example, in the breast, lung, colon, ovary, or prostate, of a siRNA able to interact with WIP1 gene or WIP 1 mRNA transcript.

The siRNA may be in the form of a vector and the vector may be a plasmid, cosmid or bacteriophage.

In still a further aspect, the present invention provides a method for determining the efficacy of a therapeutic treatment regimen in a patient, suffering from, for example, a breast cancer, a lung cancer, a prostate cancer, an ovarian cancer, or a colon cancer, etc, for example, in a clinical trial, which comprises measuring the WIP1 gene copy number in a first sample of suspected precancerous or cancer cells obtained from the patient, thereby generating an initial level. The WIP 1 gene copy number is then measured in a second sample of suspected precancerous or cancerous cells from the patient at a time following the administration of the treatment regimen, thereby generating a test level. The intial and test levels are compared, wherein a decrease in the gene copy number levels in the test level relative to the initial level indicates that the treatment regimen is effective in the patient.

In another aspect, the present invention provides a method for determining the efficacy of a therapeutic treatment regimen in a patient, suffering from, for example, a breast cancer, a lung cancer, a prostate cancer, an ovarian cancer, or a colon cancer, etc, for example, in a clinical trial, which comprises measuring at least one of the WIP 1 mRNA or WIP1 protein expression levels in a first sample of suspected precancerous or cancer cells obtained from the patient, thereby generating data for a pro-treatment level. At least one of the WIP 1 mRNA or WIP 1 protein expression levels is then measured in a second sample of suspected precancerous or cancerous cells from the patient at a time following the administration of the treatment regimen, thereby generating a test level. The pre-treatment and test levels are compared, wherein no decrease in the test level relative to the pre-treatment level indicates that the treatment regimen is not effective in the patient.

One aspect of the invention is to provide an isolated WIP1 gene amplicon, wherein the amplicon comprises more than one copy of a polynucleotide selected from: (a) a polynucleotide encoding the polypeptide set forth in SEQ ID NO : 2; (b) a polynucleotide set forth in SEQ ID NO :1; (c) a polynucleotide having at least about 90% sequence identity to the polynucleotide of (a) or (b); and (d) a polynucleotide that is overexpressed in tumour cells having at least about 90% sequence identity to the polynucleotide of (a) or (b).

WIP1 activities may be modulated by contacting a biological subject from a region that is suspected to be precancerous or cancerous with a modulator of the WIP 1 protein, wherein the modulator is, for example, a small molecule. The modulator may partially or completely inhibit transcription of WIP1.

Unless otherwise defined, all technical and scientific terms used herein in their various grammatical forms have the same meaning as commonly understood by one of ordinary skill in the art to which this invention belongs. Although methods and materials similar to those described herein can be used in the practice or testing of the present invention, the preferred methods and materials are described below. All publications, patent applications, patents, database records, for example, those in SWISS-PROT, GENBANK, EMBL, *etc.,* and other references and citations mentioned herein are incorporated by reference in their entirety. In case of conflict, the present specification, including definitions, will control. In addition, the materials, methods, and examples are illustrative only and are not limiting.

Further features, objects, and advantages of the present invention are apparent in the claims and the detailed description that follows. It should be understood, however, that the detailed description and the specific examples, while indicating preferred aspects of the invention, are given by way of illustration only, since various changes and modifications within the scope of the invention will become apparent to those skilled in the art from this detailed description.

### BRIEF DESCRIPTION OF THE DRAWINGS

**Figure 1****.** Figure shows a schematic representation of the possible interactions between WIP1 and p53,p38 in apoptosis and tumorigenesis in response to stress stimuli.
**Figure 2****.** Figure shows the epicenter mapping of 17q23 amplicon which includes WIP1 locus. The number of DNA copies for each sample is plotted on the Y-axis, and the X-axis corresponds to nucleotide position based on Human Genome Project working draft sequence I(http://genome.ucsc.edu/goldenPath/aug2001Tracks.html). The DNA copy numbers were evaluated using Q-PCR and fluorogenic Taqman probes were designed based on ESTs or BAC sequences. The markers were ordered on the basis of their physical presence on the BACs.
**Figure 3****.** Detection of endogenous WIP1 protein in MCF7 and retro-virus infected stable lines. WIP1 protein levels in mouse embryonic fibroblast C8 cells stably transfected with vector alone (pLPC) or WIP1, and breast cancer cell line MCF7 were measured by Western blot.
**Figure 4****.** Assays for oncogenic function of PAT1 and WIP1 genes.
   **4a:** WIP1 overexpression significantly attenuated apoptosis induced by serum-starvation. The number of viable cells after 48 hours of incubation in the presence of the indicated serum concentration is depicted. The empty pLPC vector (white bars), pLPC-WIP1 (dark gray bars), and pLPC-PAT1 (stippled bars) were introduced by retroviral transfection into primary mouse embryo fibroblasts transformed with E1A and RAS. These cells undergo apoptosis when starved for serum. Following selection in puromycin, 1 x 10⁶ transfected cells were plated in triplicate onto 35 mm plates. After a 16-hour incubation in serum-free medium, the cells were harvested and then cultured for 48 hours in Delbecco's Modified Eagle Medium (DMEM) with the indicated concentration of fetal bovine serum. The number of viable cells were determined using trypan blue exclusion and a hemacytometer.
   **4b:** WIP1 cooperated with mutationally activated RAS to transform primary mouse fibroblasts. A typical transformed foci of mouse embryo fibroblasts that had been infected with retroviral constructs containing WIP1 and mutationally activated RAS is depicted along with representative areas of surviving cells following infection with either the RAS or WIP1 vectors alone. Semi-confluent 100-mm dishes of primary mouse embryo fibroblasts were transfected with pLPC-derived vectors, split 1:3, and selected with puromycin for 4 days. After an additional 3 weeks of incubation, all colonies and areas of growth in plates containing cells infected with either the WIP1 or RAS vectorshad significantly receded, wheras WIP1/RAS co-transfectants formed 5 to 10 highly transformed foci. These findings were observed in two separate experiments. Four such foci were cloned and determined to overexpress WIP 1.

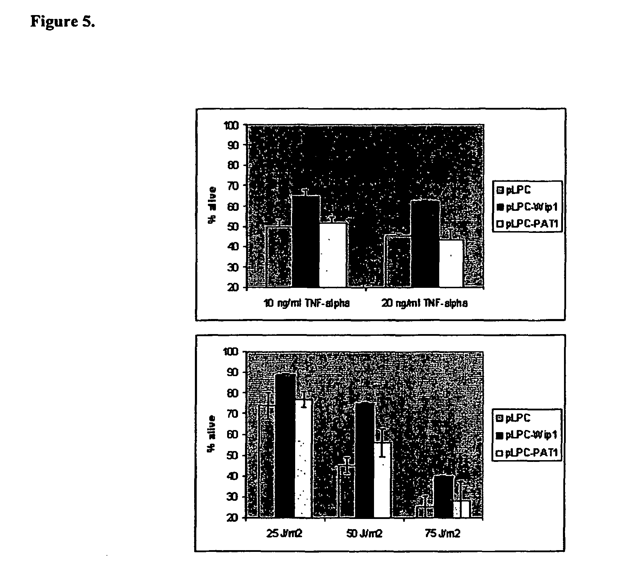

**Figure 5**. Expression of WIP1 protects TNF-α and UV induced apoptosis . Cells overexpression of WIPI attenuates TNF-α and UV induced apoptosis. C8 cells which is derived from p53+/+ mouse embryonic fibroblast that is immortalized with E1a and Ras oncogene, were stably infected with retrovirus containing just vector alone (pLPC) or WIP1 (pLPC-WIP1) or PAT1 (pLPC-PAT1). For TNF-α induced apoptosis, the medium were supplemented with 10 or 20 ng/ml TNF-α, the number of viable cells were determined using trypan blue exclusion and a hemacytometer. For UV induced apoptosis, the cells were treated with 25 J/m2, 50 J/m2, or 75 J/m2 UV radiation, the cell death was assessed by counting viable cells using trypan blue exclusion and a hemacytometer.

### DETAILED DESCRIPTION OF THE INVENTION

The present invention provides methods and compositions for the diagnosis, prevention, and treatment of tumors and cancers, for example, a breast cancer, a lung cancer, a prostate cancer, an ovarian cancer, or a colon cancer, *etc.,* in mammals, for example, humans. The invention is based on the findings of novel traits of the WIP1 genes, a stress-inducible gene that encodes a protein phosphatase type 2C (PP2C) in cancer cells. The WIP1 genes and their expressed protein products can thus be used diagnostically or as targets for therapy; and, they can also be used to identify compounds useful in the diagnosis, prevention, and therapy of tumors and cancers (for example, breast cancer, lung cancer, prostate cancer, ovarian cancer, colon cancer, *etc*).

The present invention, for the first time, provides an isolated amplified WIP1 gene. This invention also provides that the WIP1 gene is frequently amplified and overexpressed in tumor cells, for example, human breast, lung, ovarian, colon, or prostate tumors.

### Definitions:

A **"cancer"** in an animal refers to the presence of cells possessing characteristics typical of cancer-causing cells, for example, uncontrolled proliferation, loss of specialized functions, immortality, significant metastatic potential, rapid growth and proliferation rate, and certain characteristic morphology and cellular markers. In some circumstances, cancer cells will be in the form of a tumor, such cells may exist locally within an animal, or circulate in the blood stream as independent cells, for example, leukemic cells.

The phrase **"detecting a cancer" or** **"diagnosing a cancer"** refers to determining the presence or absence of cancer or a precancerous condition in an animal. "Detecting a cancer" also can refer to obtaining indirect evidence regarding the likelihood of the presence of precancerous or cancerous cells in the animal or assessing the predisposition of a patient to the development of a cancer. Detecting a cancer can be accomplished using the methods of this invention alone, in combination with other methods, or in light of other information regarding the state of health of the animal.

A **"tumor,"** as used herein, refers to all neoplastic cell growth and proliferation, whether malignant or benign, and all precancerous and cancerous cells and tissues.

The term **"precancerous"** refers to cells or tissues having characteristics relating to changes that may lead to malignancy or cancer. Examples include adenomatous growths in breast, lung, colon, ovarian, or prostate tissues, or conditions, for example, dysplastic nevus syndrome, a precursor to malignant melanoma of the skin. Examples also include, abnormal neoplastic, in addition to dysplastic nevus syndromes, polyposis syndromes, prostatic dysplasia, and other such neoplasms, whether the precancerous lesions are clinically identifiable or not.

A **"differentially expressed gene transcript",** as used herein, refers to a gene, including an oncogene, transcript that is found in different numbers of copies in different cell or tissue types of an organism having a tumor or cancer, for example, breast cancer, lung cancer, colon cancer, ovarian cancer, or prostate cancer, compared to the numbers of copies or state of the gene transcript found in the cells of the same tissue in a healthy organism, or in the cells of the same tissue in the same organism. Multiple copies of gene transcripts may be found in an organism having the tumor or cancer, while only one, or significantly fewer copies, of the same gene transcript are found in a healthy organism or healthy cells of the same tissue in the same organism, or vice-versa.

A "differentially expressed gene," can be a target, fingerprint, or pathway gene. For example, a **"fingerprint gene",** as used herein, refers to a differentially expressed gene whose expression pattern can be used as a prognostic or diagnostic marker for the evaluation of tumors and cancers, or which can be used to identify compounds useful for the treatment of tumors and cancers, for example, breast or lung cancer. For example, the effect of a compound on the fingerprint gene expression pattern normally displayed in connection with tumors and cancers can be used to evaluate the efficacy of the compound as a tumor and cancer treatment, or can be used to monitor patients undergoing clinical evaluation for the treatment of tumors and cancer.

A **"fingerprint pattern",** as used herein, refers to a pattern generated when the expression pattern of a series (which can range from two up to all the fingerprint genes that exist for a given state) of fingerprint genes is determined. A fingerprint pattern may also be referred to as an **"expression profile".** A fingerprint pattern or expression profile can be used in the same diagnostic, prognostic, and compound identification methods as the expression of a single fingerprint gene.

A **"target gene",** as used herein, refers to a differentially expressed gene in which modulation of the level of gene expression or of gene product activity prevents and/or ameliorates tumor and cancer, for example, breast cancer, symptoms. Thus, compounds that modulate the expression of a target gene, the target genes, or the activity of a target gene product can be used in the diagnosis, treatment or prevention of tumors and cancers. A particular target gene of the present invention is the WIP1 gene.

In general, a **"gene**" is a region on the genome that is capable of being transcribed to an RNA that either has a regulatory function, a catalytic function, and/or encodes a protein. A gene typically has introns and exons, which may organize to produce different RNA splice variants that encode alternative versions of a mature protein. The skilled artisan will appreciate that the present invention encompasses all WIP1-encoding transcripts that may be found, including splice variants, allelic variants and transcripts that occur because of alternative promoter sites or alternative poly-adenylation sites. A **"full-length"** gene or RNA therefore encompasses any naturally occurring splice variants, allelic variants, other alternative transcripts, splice variants generated by recombinant technologies which bear the same function as the naturally occurring variants, and the resulting RNA molecules. A **"fragment"** of a gene, including an oncogene, can be any portion from the gene, which may or may not represent a functional domain, for example, a catalytic domain, a DNA binding domain, etc. A fragment may preferably include nucleotide sequences that encode for at least 25 contiguous amino acids, and preferably at least about 30, 40, 50, 60, 65, 70, 75 or more contiguous amino acids or any integer thereabout or therebetween.

**"Pathway genes",** as used herein, are genes that encode proteins or polypeptides that interact with other gene products involved in tumors and cancers. Pathway genes also can exhibit target gene and/or fingerprint gene characteristics.

A **"detectable"** RNA expression level, as used herein, means a level that is detectable by standard techniques currently known in the art or those that become standard at some future time, and include for example, differential display, RT (reverse transcriptase)-coupled polymerase chain reaction (PCR), Northern Blot, and/or RNase protection analyses. The degree of differences in expression levels need only be large enough to be visualized or measured via standard characterization techniques, for example, any of the above.

The nucleic acid molecules of the invention, for example, the WIP1 gene or its subsequences, can be inserted into a vector, as described below, which will facilitate expression of the insert. The nucleic acid molecules and the polypeptides they encode can be used directly as diagnostic or therapeutic agents, or can be used (directly in the case of the polypeptide or indirectly in the case of a nucleic acid molecule) to generate antibodies that, in turn, are clinically useful as a therapeutic or diagnostic agent. Accordingly, vectors containing the nucleic acid of the invention, cells transfected with these vectors, the polypeptides expressed, and antibodies generated against either the entire polypeptide or an antigenic fragment thereof, are among the aspects of the invention.

As used herein, the term **"transformed cell"** means a cell into which (or into an ancestor of which) a nucleic acid molecule encoding a polypeptide of the invention has been introduced, by means of, for example, recombinant DNA techniques or viruses.

A **"structural gene"** is a DNA sequence that is transcribed into messenger RNA (mRNA) which is then translated into a sequence of amino acids characteristic of a specific polypeptide.

An **"isolated DNA molecule"** is a fragment of DNA that has been separated from the chromosomal or genomic DNA of an organism. Isolation also is defined to connote a degree of separation from original source or surroundings. For example, a cloned DNA molecule encoding an avidin gene is an isolated DNA molecule. Another example of an isolated DNA molecule is a chemically-synthesized DNA molecule, or enzymatically-produced cDNA, that is not integrated in the genomic DNA of an organism. Isolated DNA molecules can be subjected to procedures known in the art to remove contaminants such that the DNA molecule is considered purified, that is towards a more homogeneous state.

**"Complementary DNA"** (cDNA) is a single-stranded DNA molecule that is formed from an mRNA template by the enzyme reverse transcriptase. Typically, a primer complementary to portions of the mRNA is employed for the initiation of reverse transcription. Those skilled in the art also use the term "cDNA" to refer to a double-stranded DNA molecule that comprises such a single-stranded DNA molecule and its complementary DNA strand.

The term **"expression"** refers to the biosynthesis of a gene product. For example, in the case of a structural gene, expression involves transcription of the structural gene into mRNA and the translation of mRNA into one or more polypeptides.

The term **"amplification"** refers to amplification, duplication, multiplication, or multiple expression of nucleic acids or a gene, *in vivo* or *in vitro,* yielding about 2.5 fold or more copies. For example, amplification of the WIP1 gene resulting in a copy number greater than or equal to 2.5 is deemed to have been amplified.

The term **"amplicon"** refers to an amplification product containing one or more genes, which can be isolated from a precancerous or a cancerous cell or a tissue. WIP1 amplicon is a result of amplification, duplication, multiplication, or multiple expression of nucleic acids or a gene, *in vivo* or *in vitro.* "Amplicon", as defined herein, also include a completely or partially amplified WIP1 gene. For example, an amplicon comprising a polynucleotide having at least about 90% sequence identity to SEQ ID NO:1 or any fragment thereof.

A **"cloning vector"** is a nucleic acid molecule, for example, a plasmid, cosmid, or bacteriophage that has the capability of replicating autonomously in a host cell. Cloning vectors typically contain (i) one or a small number of restriction endonuclease recognition sites at which foreign DNA sequences can be inserted in a determinable fashion without loss of an essential biological function of the vector, and (ii) a marker gene that is suitable for use in the identification and selection of cells transformed with the cloning vector. Marker genes include genes that provide tetracycline resistance or ampicillin resistance, for example.

An **"expression vector"** is a nucleic acid construct, generated recombinantly or synthetically, bearing a series of specified nucleic acid elements that enable transcription of a particular gene in a host cell. Typically, gene expression is placed under the control of certain regulatory elements, including constitutive or inducible promoters, tissue-preferred regulatory elements, and enhancers. Such a gene is said to be "operably linked to" or "operatively linked to" the regulatory elements, which means that the regulatory elements control the expression of the gene.

A **"recombinant host**" may be any prokaryotic or eukaryotic cell that contains either a cloning vector or expression vector. This term also includes those prokaryotic or eukaryotic cells that have been genetically engineered to contain the cloned gene(s) in the chromosome or genome of the host cell.

In eukaryotes, RNA polymerase II catalyzes the transcription of a structural gene to produce mRNA. A DNA molecule can be designed to contain an RNA polymerase II template in which the RNA transcript has a sequence that is complementary to that of a preferred mRNA. The RNA transcript is termed an **"antisense RNA".** Antisense RNA molecules inhibit mRNA expression. With respect to a first nucleic acid molecule, a second DNA molecule having a sequence that is complementary to the sequence of the first molecule or the portions thereof is referred to as the **"****antisense DNA"** of the first molecule.

The term **"****operably linked"** is used to describe the connection between regulatory elements and a gene or its coding region. That is, gene expression is typically placed under the control of certain regulatory elements, including constitutive or inducible promoters, tissue-specific regulatory elements, and enhancers. Such a gene is said to be "operably linked to" or "operatively linked to" the regulatory elements.

**"Sequence homology"** is used to describe the sequence relationships between two or more nucleic acids, polynucleotides, proteins, or polypeptides, and is understood in the context of and in conjunction with the terms including: (a) reference sequence, (b) comparison window, (c) sequence identity, (d) percentage of sequence identity, and (e) substantial identity or "homologous."
(a) A "**reference sequence"** is a defined sequence used as a basis for sequence comparison. A reference sequence may be a subset of or the entirety of a specified sequence; for example, a segment of a full-length cDNA or gene sequence, or the complete cDNA or gene sequence. For polypeptides, the length of the reference polypeptide sequence will generally be at least about 16 amino acids, preferably at least about 20 amino acids, more preferably at least about 25 amino acids, and most preferably about 35 amino acids, about 50 amino acids, or about 100 amino acids. For nucleic acids, the length of the reference nucleic acid sequence will generally be at least about 50 nucleotides, preferably at least about 60 nucleotides, more preferably at least about 75 nucleotides, and most preferably about 100 nucleotides or about 300 nucleotides.
(b) A **"comparison window"** includes reference to a contiguous and specified segment of a polynucleotide sequence, wherein the polynucleotide sequence may be compared to a reference sequence and wherein the portion of the polynucleotide sequence in the comparison window may comprise additions, substitutions, or deletions (i.e., gaps) compared to the reference sequence (which does not comprise additions, substitutions, or deletions) for optimal alignment of the two sequences. Generally, the comparison window is at least 20 contiguous nucleotides in length, and optionally can be 30, 40, 50, 100, or longer. Those of skill in the art understand that to avoid a misleadingly high similarity to a reference sequence due to inclusion of gaps in the polynucleotide sequence a gap penalty is typically introduced and is subtracted from the number of matches.
   Methods of alignment of sequences for comparison are well-known in the art. Optimal alignment of sequences for comparison may be conducted by the local homology algorithm of Smith and Waterman, Adv. Appl. Math. 2: 482 (1981); by the homology alignment algorithm of Needleman and Wunsch, J. Mol. Biol. 48: 443 (1970); by the search for similarity method of Pearson and Lipman, Proc. Natl. Acad Sci. 8: 2444 (1988); by computerized implementations of these algorithms, including, but not limited to: CLUSTAL in the PC/Gene program by Intelligenetics, Mountain View, California, GAP, BESTFIT, BLAST, FASTA, and TFASTA in the Wisconsin Genetics Software Package, Genetics Computer Group (GCG), 7 Science Dr., Madison, Wisconsin, USA; the CLUSTAL program is well described by Higgins and Sharp, Gene 73: 237-244 (1988); Higgins and Sharp, CABIOS : 11-13 (1989); Corpet, et al., Nucleic Acids Research 16: 881-90 (1988); Huang, et al., Computer Applications in the Biosciences 8: 1-6 (1992), and Pearson, et al., Methods in Molecular Biology 24: 7-331 (1994). The BLAST family of programs which can be used for database similarity searches includes: BLASTN for nucleotide query sequences against nucleotide database sequences; BLASTX for nucleotide query sequences against protein database sequences; BLASTP for protein query sequences against protein database sequences; TBLASTN for protein query sequences against nucleotide database sequences; and TBLASTX for nucleotide query sequences against nucleotide database sequences. See, Current Protocols in Molecular Biology, Chapter 19, Ausubel, et al., Eds., Greene Publishing and Wiley-Interscience, New York (1995). New versions of the above programs or new programs altogether will undoubtedly become available in the future, and can be used with the present invention.
   Unless otherwise stated, sequence identity/similarity values provided herein refer to the value obtained using the BLAST 2.0 suite of programs using default parameters. Altschul et al., Nucleic Acids Res. 2:3389-3402 (1997). It is to be understood that default settings of these parameters can be readily changed as needed in the future.
   As those ordinary skilled in the art will understand, BLAST searches assume that proteins can be modeled as random sequences. However, many real proteins comprise regions of nonrandom sequences which may be homopolymeric tracts, short-period repeats, or regions enriched in one or more amino acids. Such low-complexity regions may be aligned between unrelated proteins even though other regions of the protein are entirely dissimilar. A number of low-complexity filter programs can be employed to reduce such low-complexity alignments. For example, the SEG (Wooten and Federhen, Comput. Chem., 17:149-163 (1993)) and XNU (Claverie and States, Comput. Chem., 17:191-1 (1993)) low-complexity filters can be employed alone or in combination.
(c) "**Sequence identity"** or **"identity"** in the context of two nucleic acid or polypeptide sequences includes reference to the residues in the two sequences which are the same when aligned for maximum correspondence over a specified comparison window, and can take into consideration additions, deletions and substitutions. When percentage of sequence identity is used in reference to proteins it is recognized that residue positions which are not identical often differ by conservative amino acid substitutions, where amino acid residues are substituted for other amino acid residues with similar chemical properties (for example, charge or hydrophobicity) and therefore do not change the functional properties of the molecule. Where sequences differ in conservative substitutions, the percent sequence identity may be adjusted upwards to correct for the conservative nature of the substitution. Sequences which differ by such conservative substitutions are said to have sequence similarity or similarity. Means for making this adjustment are well-known to those of skill in the art. Typically this involves scoring a conservative substitution as a partial rather than a full mismatch, thereby increasing the percentage sequence identity. Thus, for example, where an identical amino acid is given a score of 1 and a non-conservative substitution is given a score of zero, a conservative substitution is given a score between zero and 1. The scoring of conservative substitutions is calculated, for example, according to the algorithm of Meyers and Miller, Computer Applic. Biol. Sci., 4: 11-17 (1988) for example, as implemented in the program PC/GENE (Intelligenetics, Mountain View, California, USA).
(d) **"Percentage of sequence identity"** means the value determined by comparing two optimally aligned sequences over a comparison window, wherein the portion of the polynucleotide sequence in the comparison window may comprise additions, substitutions, or deletions (i.e., gaps) as compared to the reference sequence (which does not comprise additions, substitutions, or deletions) for optimal alignment of the two sequences. The percentage is calculated by determining the number of positions at which the identical nucleic acid base or amino acid residue occurs in both sequences to yield the number of matched positions, dividing the number of matched positions by the total number of positions in the window of comparison and multiplying the result by 100 to yield the percentage of sequence identity.
(e) (i) The term **"substantial identity"** or **"homologous"** in their various grammatical forms means that a polynucleotide comprises a sequence that has a desired identity, for example, at least 60% identity, preferably at least 70% sequence identity, more preferably at least 80%, still more preferably at least 90% and most preferably at least 95%, compared to a reference sequence using one of the alignment programs described using standard parameters. One of skill will recognize that these values can be appropriately adjusted to determine corresponding identity of proteins encoded by two nucleotide sequences by taking into account codon degeneracy, amino acid similarity, reading frame positioning and the like. Substantial identity of amino acid sequences for these purposes normally means sequence identity of at least 60%, more preferably at least 70%, 80%, 90%, and most preferably at least 95%.

Another indication that nucleotide sequences are substantially identical is if two molecules hybridize to each other under stringent conditions. However, nucleic acids which do not hybridize to each other under stringent conditions are still substantially identical if the polypeptides which they encode are substantially identical. This may occur, for example, when a copy of a nucleic acid is created using the maximum codon degeneracy permitted by the genetic code. One indication that two nucleic acid sequences are substantially identical is that the polypeptide which the first nucleic acid encodes is immunologically cross reactive with the polypeptide encoded by the second nucleic acid, although such cross-reactivity is not required for two polypeptides to be deemed substantially identical.
(e) (ii) The terms **"substantial identity"** or **"homologous"** in their various grammatical forms in the context of a peptide indicates that a peptide comprises a sequence that has a desired identity, for example, at least 60% identity, preferably at least 70% sequence identity to a reference sequence, more preferably 80%, still more preferably 85%, most preferably at least 90% or 95% sequence identity to the reference sequence over a specified comparison window. Preferably, optimal alignment is conducted using the homology alignment algorithm of Needleman and Wunsch, J. Mol. Biol. 48: 443 (1970). An indication that two peptide sequences are substantially identical is that one peptide is immunologically reactive with antibodies raised against the second peptide, although such cross-reactivity is not required for two polypeptides to be deemed substantially identical. Thus, a peptide is substantially identical to a second peptide, for example, where the two peptides differ only by a conservative substitution. Peptides which are "substantially similar" share sequences as noted above except that residue positions which are not identical may differ by conservative amino acid changes. Conservative substitutions typically include, but are not limited to, substitutions within the following groups: glycine and alanine; valine, isoleucine, and leucine; aspartic acid and glutamic acid; asparagine and glutamine; serine and threonine; lysine and arginine; and phenylalanine and tyrosine.

The term **"WIP1"** refers to WIP1 nucleic acid (DNA and RNA), protein (or polypeptide), and can include their polymorphic variants, alleles, mutants, and interspecies homologs that have (i) substantial nucleotide sequence homology with the nucleotide sequence of the GenBank entry AAB61637 (human WIP1); or (ii) at least 65% sequence homology with the amino acid sequence of the SWISS-PROT record 015297 (Protein Phosphatase 2C δ Isoform); or (iii) substantial nucleotide sequence homology with the nucleotide sequence as set forth in SEQ ID NO:1; or (iv) substantial sequence homology with the encoded amino acid sequence.

WIP1 polynucleotide or polypeptide sequences are typically from a mammal including, but not limited to, human, rat, mouse, hamster, cow, pig, horse, sheep, or any mammal. A "WIP1 polynucleotide" and a "WIP1 polypeptide," may be either naturally occurring, recombinant, or synthetic (for example, via chemical synthesis).

The **"level of WIP1 mRNA"** in a biological sample refers to the amount of mRNA transcribed from a WIP1 gene that is present in a cell or a biological sample. The mRNA generally encodes a WIP1 protein, often fully functional, although mutations or deletions may be present that alter or eliminate the function of the encoded protein. A "level of WIP1 mRNA" need not be quantified, but can simply be detected, for example, via a subjective, visual detection by a human, with or without comparison to a level from a control sample or a level expected of a control sample.

The **"level of WIP1 protein or polypeptide"** in a biological sample refers to the amount of polypeptide translated from a WIP1 mRNA that is present in a cell or biological sample. The polypeptide may or may not have WIP1 protein activity. A "level of WIP1 protein" need not be quantified, but can simply be detected, for example, via a subjective, visual detection by a human, with or without comparison to a level from a control sample or a level expected of a control sample.

A **"full length"** WIP1 protein or nucleic acid refers to a WIP1 polypeptide or polynucleotide sequence, or a variant thereof, that contains all of the elements normally contained in one or more naturally occurring, wild type WIP1 polynucleotide or polypeptide sequences.

**"Biological subject"** as used herein refers to a target biological object obtained, reached, or collected *in vivo* or *in situ,* including a biological sample, for example, a cell, a tissue, an organ, or body fluid, that contains or is suspected of containing nucleic acids or polypeptides of WIP1. Such biological subjects include, but are not limited to, tissue originated in humans, mice, and rats. Biological subjects may also include sections of the biological subject including tissues, for example, frozen sections taken for histologic purposes. A biological subject is typically of eukaryotic nature, for example, insects, protozoa, birds, fish, reptiles, and preferably a mammal, for example, rat, mouse, cow, dog, guinea pig, or rabbit, and most preferably a primate, for example, chimpanzees or humans.

**"Biological sample"** as used herein is a biological subject *in vivo* or *in situ,* including sample of biological tissue or fluid origin that contains or is suspected of containing nucleic acids or polypeptides of WIP1. Such samples include, but are not limited to, tissue isolated from humans, mice, and rats. Biological samples may also include sections of the biological sample including tissues, for example, frozen sections taken for histologic purposes. A biological sample is typically of an eukaryotic origin, for example, insects, protozoa, birds, fish, reptiles, and preferably a mammal, for example, rat, mouse, cow, dog, guinea pig, or rabbit, and most preferably a primate, for example, chimpanzees or humans.

**"Providing a biological subject"** means to obtain a biological subject *in vivo* or *in situ*, including tissue or cell sample for use in the methods described in the present invention. Most often, this will be done by removing a sample of cells from an animal, but can also be accomplished *in vivo* or *in situ* or by using previously isolated cells (for example, isolated by another person, at another time, and/or for another purpose), or by performing the methods of this invention *in vivo.*

A "**control sample**" refers to a sample of biological material representative of healthy, cancer-free animals. The level of WIP1 or WIP1 gene copy number in a control sample is desirably typical of the general population of normal, cancer-free animals of the same species. This sample either can be collected from an animal for the purpose of being used in the methods described in the present invention or, it can be any biological material representative of normal, cancer-free animals obtained for other reasons but nonetheless suitable for use in the methods of this invention. A control sample can also be obtained from normal tissue from the animal that has cancer or is suspected of having cancer. A control sample also can refer to a given level of WIP1 representative of the cancer-free population, that has been previously established based on measurements from normal, cancer-free animals. Alternatively, a biological control sample can refer to a sample that is obtained from a different individual or be a normalized value based on baseline values found in a population. Further, a control sample can be defined by a specific age, sex, ethnicity or other demographic parameters. In some situations, the control is implicit in the particular measurement. For example, a detection method that can only detect WIP1 or WIP1 gene copy number when a level higher than that typical of a normal, cancer-free animal is present, for example, an immunohistochemical assay, is considered to be assessing the WIP1 level in or WIP 1 gene copy number comparison to the control level or WIP 1 gene copy number, as the control level or the copy number is natural and known in the assay.

**"Data"** refers to information obtained that relates to "Biological Sample" or "Control Sample", as described above, wherein the information is applied in generating a test level for diagnostics, prevention, monitoring or therapeutic use. The present invention relates to methods for comparing and compiling data wherein the data is stored in electronic or paper formats. Electronic format can be selected from the group consisting of electronic mail, disk, compact disk (CD), digital versatile disk (DVD), memory card, memory chip, ROM or RAM, magnetic optical disk, tape, video, video clip, microfilm, internet, shared network, shared server and the like; wherein data is displayed, transmitted or analyzed via electronic transmission, video display, telecommunication, or by using any of the above stored formats; wherein data is compared and compiled at the site of sampling specimens or at a location where the data is transported following a process as described above.

**"Overexpression"** of a WIP1 gene or an "increased," or "elevated," level of a WIP1 polynucleotide or protein refers to a level of WIP1 polynucleotide or polypeptide that, in comparison with a control level of WIP1, is detectably higher. Comparison may be carried out by statistical analyses on numeric measurements of the expression; or, it may be done through visual examination of experimental results by qualified researchers.

A level of WIP polypeptide or polynucleotide that is **"expected"** in a control sample refers to a level that represents a typical, cancer-free sample, and from which an elevated, or diagnostic, presence of WIP polypeptide or polynucleotide can be distinguished. Preferably, an "expected" level will be controlled for such factors as the age, sex, medical history, *etc.* of the mammal, as well as for the particular biological subject being tested.

The phrase **"functional effects"** in the context of an assay or assays for testing compounds that modulate WIP1 activity includes the determination of any parameter that is indirectly or directly under the influence of WIP1, for example, a functional, physical, or chemical effect, for example, the protease activity, the ability to induce gene amplification or overexpression in cancer cells, and to aggravate cancer cell proliferation. "Functional effects" include *in vitro, in vivo,* and *ex vivo* activities.

**"Determining the functional effect"** refers to assaying for a compound that increases or decreases a parameter that is indirectly or directly under the influence of WIP1, for example, functional, physical, and chemical effects. Such functional effects can be measured by any means known to those skilled in the art, for example, changes in spectroscopic characteristics (for example, fluorescence, absorbance, refractive index), hydrodynamic (for example, shape), chromatographic, or solubility properties for the protein, measuring inducible markers or transcriptional activation of WIP1; measuring binding activity or binding assays, for example, substrate binding, and measuring cellular proliferation; measuring signal transduction; or measuring cellular transformation.

**"Inhibitors," "activators," "modulators"** and **"regulators"** refer to molecules that activate, inhibit, modulate and/or regulate an identified function. For example, referring to WIP1 activity, such molecules may be identified using *in vitro* and *in vivo* assays of WIP1. Inhibitors are compounds that partially or totally block WIP 1 activity, decrease, prevent, or delay its activation, or desensitize its cellular response. This may be accomplished by binding to WIP1 proteins directly or via other intermediate molecules. An antagonist of WIP1 is considered to be such an inhibitor. Activators are compounds that bind to WIP1 protein directly or via other intermediate molecules, thereby increasing or enhancing its activity, stimulating or accelerating its activation, or sensitizing its cellular response. An agonist of WIP1 is considered to be such an activator. A modulator can be an inhibitor or activator. A modulator may or may not bind WIP1 or its protein directly; it affects or changes the activity or activation of WIP1 or the cellular sensitivity to WIP1. A modulator also may be a compound, for example, a small molecule, that inhibits transcription of WIP1 mRNA.

The group of inhibitors, activators and modulators of this invention also includes genetically modified versions of WIP1, for example, versions with altered activity. The group thus is inclusive of the naturally occurring protein as well as synthetic ligands, antagonists, agonists, antibodies, small chemical molecules and the like.

**"Assays for inhibitors, activators, or modulators"** refer to experimental procedures including, for example, expressing WIP1 *in vitro,* in cells, applying putative inhibitor, activator, or modulator compounds, and then determining the functional effects on WIP1 activity, as described above. Samples that contain or are suspected of containing WIP1 are treated with a potential activator, inhibitor, or modulator. The extent of activation, inhibition, or change is examined by comparing the activity measurement from the samples of interest to control samples. A threshold level is established to assess activation or inhibition. For example, inhibition of a WIP1 polypeptide is considered achieved when the WIP1 activity value relative to the control is 80% or lower. Similarly, activation of a WIP1 polypeptide is considered achieved when the WIP 1 activity value relative to the control is two or more fold higher.

The terms **"isolated," "purified,"** or **"biologically pure"** refer to material that is free to varying degrees from components which normally accompany it as found in its native state. "Isolate" denotes a degree of separation from original source or surroundings. "Purify" denotes a degree of separation that is higher than isolation. A "purified" or "biologically pure" protein is sufficiently free of other materials such that any impurities do not materially affect the biological properties of the protein or cause other adverse consequences. That is, a nucleic acid or peptide of this invention is purified if it is substantially free of cellular material, viral material, or culture medium when produced by recombinant DNA techniques, or chemical precursors or other chemicals when chemically synthesized. Purity and homogeneity are typically determined using analytical chemistry techniques, for example, polyacrylamide gel electrophoresis or high performance liquid chromatography. The term "purified" can denote that a nucleic acid or protein gives rise to essentially one band in an electrophoretic gel. For a protein that can be subjected to modifications, for example, phosphorylation or glycosylation, different modifications may give rise to different isolated proteins, which can be separately purified. Various levels of purity may be applied as needed according to this invention in the different methodologies set forth herein; the customary purity standards known in the art may be used if no standard is otherwise specified.

An **"****isolated nucleic acid molecule"** can refer to a nucleic acid molecule, depending upon the circumstance, that is separated from the 5' and 3' coding sequences of genes or gene fragments contiguous in the naturally occurring genome of an organism. The term "isolated nucleic acid molecule" also includes nucleic acid molecules which are not naturally occurring, for example, nucleic acid molecules created by recombinant DNA techniques.

**"Nucleic acid"** refers to deoxyribonucleotides or ribonucleotides and polymers thereof in either single- or double-stranded form. The term encompasses nucleic acids containing known nucleotide analogs or modified backbone residues or linkages, which are synthetic, naturally occurring, and non-naturally occurring, which have similar binding properties as the reference nucleic acid, and which are metabolized in a manner similar to the reference nucleotides. Examples of such analogs include, without limitation, phosphorothioates, phosphoramidates, methyl phosphonates, chiral methyl phosphonates, 2-O-methyl ribonucleotides, and peptide-nucleic acids (PNAs).

Unless otherwise indicated, a particular nucleic acid sequence also implicitly encompasses conservatively modified variants thereof (for example, degenerate codon substitutions) and complementary sequences, as well as the sequence explicitly indicated. Specifically, degenerate codon substitutions may be achieved by generating sequences in which the third position of one or more selected (or all) codons is substituted with suitable mixed base and/or deoxyinosine residues (Batzer et al., Nucleic Acid Res. 19:081 (1991); Ohtsuka et al., J. Biol. Chem. 260:2600-2608 (1985); Rossolini et al., Mol. Cell. Probes 8:91-98 (1994)). The term nucleic acid is used interchangeably with gene, cDNA, mRNA, oligonucleotide, and polynucleotide.

A **"host cell"** is a naturally occurring cell or a transformed cell that contains an expression vector and supports the replication or expression of the expression vector. Host cells may be cultured cells, explants, cells *in vivo,* and the like. Host cells may be prokaryotic cells, for example, *E*. *coli,* or eukaryotic cells, for example, yeast, insect, amphibian, or mammalian cells, for example, CHO, HeLa, and the like.

The term **"amino acid"** refers to naturally occurring and synthetic amino acids, as well as amino acid analogs and amino acid mimetics that function in a manner similar to the naturally occurring amino acids. Naturally occurring amino acids are those encoded by the genetic code, as well as those amino acids that are later modified, for example, hydroxyproline, γ-carboxyglutamate, and O-phosphoserine, phosphotheorine. **"Amino acid analogs**" refer to compounds that have the same basic chemical structure as a naturally occurring amino acid, *i.e.,* a carbon that is bound to a hydrogen, a carboxyl group, an amino group, and an R group, for example, homoserine, norleucine, methionine sulfoxide, methionine methyl sulfonium. Such analogs have modified R groups (for example, norleucine) or modified peptide backbones, but retain the same basic chemical structure as a naturally occurring amino acid. **"****Amino acid mimetics"** refers to chemical compounds that have a structure that is different from the general chemical structure of an amino acid, but that function in a manner similar to a naturally occurring amino acid. Amino acids and analogs are well known in the art.

Amino acids may be referred to herein by either their commonly known three letter symbols or by the one-letter symbols recommended by the IUPAC-IUB Biochemical Nomenclature Commission. Nucleotides, likewise, may be referred to by their commonly accepted single-letter codes.

**"Conservatively modified variants"** apply to both amino acid and nucleic acid sequences. With respect to particular nucleic acid sequences, conservatively modified variants refers to those nucleic acids which encode identical or similar amino acid sequences and include degenerate sequences. For example, the codons GCA, GCC, GCG and GCU all encode alanine. Thus, at every amino acid position where an alanine is specified, any of these codons can be used interchangeably in constructing a corresponding nucleotide sequence. The resulting nucleic acid variants are conservatively modified variants, since they encode the same protein (assuming that is the only alternation in the sequence). One skilled in the art recognizes that each codon in a nucleic acid, except for AUG (sole codon for methionine) and TGG (tryptophan), can be modified conservatively to yield a functionally-identical peptide or protein molecule.

As to amino acid sequences, one skilled in the art will recognize that substitutions, deletions, or additions to a polypeptide or protein sequence which alter, add or delete a single amino acid or a small number (typically less than ten) of amino acids is a "conservatively modified variant" where the alteration results in the substitution of an amino acid with a chemically similar amino acid. Conservative substitutions are well known in the art and include, for example, the changes of: alanine to serine; arginine to lysine; asparigine to glutamine or histidine; aspartate to glutamate; cysteine to serine; glutamine to asparigine; glutamate to aspartate; glycine to proline; histidine to asparigine or glutamine; isoleucine to leucine or valine; leucine to valine or isoleucine; lysine to arginine, glutamine, or glutamate; methionine to leucine or isoleucine; phenylalanine to tyrosine, leucine or methionine; serine to threonine; threonine to serine; tryptophan to tyrosine; tyrosine to tryptophan or phenylalanine; valine to isoleucine or leucine.

The terms **"protein"**, "**peptide"** and "**polypeptide**" are used herein to describe any chain of amino acids, regardless of length or post-translational modification (for example, glycosylation or phosphorylation). Thus, the terms can be used interchangeably herein to refer to a polymer of amino acid residues. The terms also apply to amino acid polymers in which one or more amino acid residue is an artificial chemical mimetic of a corresponding naturally occurring amino acid. Thus, the term "polypeptide" includes full-length, naturally occurring proteins as well as recombinantly or synthetically produced polypeptides that correspond to a full-length naturally occurring protein or to particular domains or portions of a naturally occurring protein. The term also encompasses mature proteins which have an added amino-terminal methionine to facilitate expression in prokaryotic cells.

The polypeptides of the invention can be chemically synthesized or synthesized by recombinant DNA methods; or, they can be purified from tissues in which they are naturally expressed, according to standard biochemical methods of purification.

Also included in the invention are **"functional polypeptides,"** which possess one or more of the biological functions or activities of a protein or polypeptide of the invention. These functions or activities include the ability to bind some or all of the proteins which normally bind to WIP 1 protein.

The functional polypeptides may contain a primary amino acid sequence that has been modified from that considered to be the standard sequence of WIP1 described herein. Preferably these modifications are conservative amino acid substitutions, as described herein.

A **"label"** or a **"detectable moiety"** is a composition that when linked with the nucleic acid or protein molecule of interest renders the latter detectable, via spectroscopic, photochemical, biochemical, immunochemical, or chemical means. For example, useful labels include radioactive isotopes, magnetic beads, metallic beads, colloidal particles, fluorescent dyes, electron-dense reagents, enzymes (for example, as commonly used in an ELISA), biotin, digoxigenin, or haptens. A **"labeled nucleic acid or oligonucleotide probe"** is one that is bound, either covalently, through a linker or a chemical bond, or noncovalently, through ionic, van der Waals, electrostatic, hydrophobic interactions, or hydrogen bonds, to a label such that the presence of the nucleic acid or probe may be detected by detecting the presence of the label bound to the nucleic acid or probe.

As used herein a **"nucleic acid or oligonucleotide** probe" is defined as a nucleic acid capable of binding to a target nucleic acid of complementary sequence through one or more types of chemical bonds, usually through complementary base pairing, usually through hydrogen bond formation. As used herein, a probe may include natural (*i.e*., A, G, C, or T) or modified bases (7-deazaguanosine, inosine, *etc.).* In addition, the bases in a probe may be joined by a linkage other than a phosphodiester bond, so long as it does not interfere with hybridization. It will be understood by one of skill in the art that probes may bind target sequences lacking complete complementarity with the probe sequence depending upon the stringency of the hybridization conditions. The probes are preferably directly labeled with isotopes, for example, chromophores, lumiphores, chromogens, or indirectly labeled with biotin to which a streptavidin complex may later bind. By assaying for the presence or absence of the probe, one can detect the presence or absence of a target gene of interest.

The phrase **"selectively (or specifically) hybridizes to" r**efers to the binding, duplexing, or hybridizing of a molecule only to a particular nucleotide sequence under stringent hybridization conditions when that sequence is present in a complex mixture (for example, total cellular or library DNA or RNA).

The phrase **"stringent-hybridization conditions"** refers to conditions under which a probe will hybridize to its target complementary sequence, typically in a complex mixture of nucleic acids, but to no other sequences. Stringent conditions are sequence-dependent and circumstance-dependent; for example, longer sequences hybridize specifically at higher temperatures. An extensive guide to the hybridization of nucleic acids is found in Tijssen, *Techniques in Biochemistry and Molecular Biology-Hybridization with Nucleic Probes,* "Overview of principles of hybridization and the strategy of nucleic acid assays" (1993). In the context of the present invention, as used herein, the term "hybridizes under stringent conditions" is intended to describe conditions for hybridization and washing under which nucleotide sequences at least 60% homologous to each other typically remain hybridized to each other. Preferably, the conditions are such that sequences at least about 65%, more preferably at least about 70%, and even more preferably at least about 75% or more homologous to each other typically remain hybridized to each other.

Generally, stringent conditions are selected to be about 5-10°C lower than the thermal melting point (Tm) for the specific sequence at a defined ionic strength pH. The Tm is the temperature (under defined ionic strength, pH, and nucleic concentration) at which 50% of the probes complementary to the target hybridize to the target sequence at equilibrium (as the target sequences are present in excess, at TR, 50% of the probes are occupied at equilibrium). Stringent conditions will be those in which the salt concentration is less than about 1.0 M sodium ion, typically about 0.01 to 1.0 M sodium ion concentration (or other salts) at pH 7.0 to 8.3 and the temperature is at least about 30°C for short probes (for example, 10 to 50 nucleotides) and at least about 60°C for long probes (for example, greater than 50 nucleotides). Stringent conditions may also be achieved with the addition of destabilizing agents, for example, formamide. For selective or specific hybridization, a positive signal is at least two times background, preferably 10 times background hybridization.

Exemplary, non-limiting stringent hybridization conditions can be as following: 50% formamide, 5x SSC, and 1 % SDS, incubating at 42°C, or, 5x SSC, 1 SDS, incubating at 65°C, with wash in 0.2x SSC, and 0.1% SDS at 65°C. Alternative conditions include, for example, conditions at least as stringent as hybridization at 68°C for 20 hours, followed by washing in 2x SSC, 0.1% SDS, twice for 30 minutes at 55°C and three times for 15 minutes at 60°C. Another alternative set of conditions is hybridization in 6x SSC at about 45°C, followed by one or more washes in 0.2x SSC, 0.1% SDS at 50-65°C. For PCR, a temperature of about 36°C is typical for low stringency amplification, although annealing temperatures may vary between about 32°C and 48°C depending on primer length. For high stringency PCR amplification, a temperature of about 62°C is typical, although high stringency annealing temperatures can range from about 50°C to about 65°C, depending on the primer length and specificity. Typical cycle conditions for both high and low stringency amplifications include a denaturation phase of 90°C - 95°C for 30 sec. - 2 min., an annealing phase lasting 30 sec. - 2 min., and an extension phase of about 72°C for 1 - 2 min.

Nucleic acids that do not hybridize to each other under stringent conditions are still substantially identical if the polypeptides which they encode are substantially identical. This occurs, for example, when a copy of a nucleic acid is created using the maximum codon degeneracy permitted by the genetic code. In such cases, the nucleic acids typically hybridize under moderately stringent hybridization conditions. Exemplary "moderately stringent hybridization conditions" include a hybridization in a buffer of 40% formamide, I M NaCl, 1% SDS at 37°C, and a wash in 1x SSC at 45°C. A positive hybridization is at least twice background. Those of ordinary skill will readily recognize that alternative hybridization and wash conditions can be utilized to provide conditions of similar stringency.

**"Antibody"** refers to a polypeptide comprising a framework region encoded by an immunoglobulin gene or fragments thereof that specifically binds and recognizes an antigen. The recognized immunoglobulin genes include the kappa, lambda, alpha, gamma, delta, epsilon, and mu constant region genes, as well as the myriad immunoglobulin variable region genes. Light chains are classified as either kappa or lambda. Heavy chains are classified as gamma, mu, alpha, delta, or epsilon, which in turn define the immunoglobulin classes, IgG, IgM, IgA, IgD and IgE, respectively. An exemplary immunoglobulin (antibody) structural unit comprises a tetramer. Each tetramer is composed of two identical pairs of polypeptide chains, each pair having one "light" (about 2 kD) and one "heavy" chain (about 0-70 kD).

Antibodies exist, for example, as intact immunoglobulins or as a number of well-characterized fragments produced by digestion with various peptidases. While various antibody fragments are defined in terms of the digestion of an intact antibody, one of skilled in the art will appreciate that such fragments may be synthesized *de novo* chemically or via recombinant DNA methodologies. Thus, the term antibody, as used herein, also includes antibody fragments produced by the modification of whole antibodies, those synthesized *de novo* using recombinant DNA methodologies (for example, single chain Fv), humanized antibodies, and those identified using phage display libraries (see, for example, Knappik et al. J Mol Biol. 2000 296:57-86; McCafferty et al., Nature 348:2-4 (1990)), for example. For preparation of antibodies - recombinant, monoclonal, or polyclonal antibodies - any technique known in the art can be used in this invention (see, for example, Kohler & Milstein, Nature 26:49-497 (1997); Kozbor et al., Immunology Today 4: 72 (1983); Cole et al., pp. 77-96 in Monoclonal Antibodies and Cancer Therapy, Alan R. Liss, Inc. (1998)).

Techniques for the production of single chain antibodies (See U.S. Patent 4,946,778) can be adapted to produce antibodies to polypeptides of this invention. Transgenic mice, or other organisms, for example, other mammals, may be used to express humanized antibodies. Phage display technology can also be used to identify antibodies and heteromeric Fab fragments that specifically bind to selected antigens (see, for example, McCafferty et al., Nature 348:2-4 (1990); Marks et al., Biotechnology :779-783 (1992)).

An **"anti-WIP1"** antibody is an antibody or antibody fragment that specifically binds a polypeptide encoded by a WIP1 gene, cDNA, or a subsequence thereof.

The term **"immunoassay"** is an assay that utilizes the binding interaction between an antibody and an antigen. Typically, an immunoassay uses the specific binding properties of a particular antibody to isolate, target, and/or quantify the antigen.

The phrase **"specifically (or selectively) binds"** to an antibody or **"specifically (or selectively) immunoreactive with,"** when referring to a protein or peptide, refers to a binding reaction that is determinative of the presence of the protein in a heterogeneous population of proteins and other biologics. Thus, under designated immunoassay conditions, the specified antibodies bind to a particular protein at a level at least two times the background and do not substantially bind in a significant amount to other proteins present in the sample. Specific binding to an antibody under such conditions may require an antibody that is selected for its specificity for a particular protein. For example, antibodies raised to a particular WIP1 polypeptide can be selected to obtain only those antibodies that are specifically immunoreactive with the WIP1 polypeptide, respectively, and not with other proteins, except for polymorphic variants, orthologs, and alleles of the specific WIP 1 polypeptide. In addition, antibodies raised to a particular WIP1 polypeptide ortholog can be selected to obtain only those antibodies that are specifically immunoreactive with the WIP1 polypeptide ortholog, respectively, and not with other orthologous proteins, except for polymorphic variants, mutants, and alleles of the WIP1 polypeptide ortholog. This selection may be achieved by subtracting out antibodies that cross-react with desired WIP1 molecule, as appropriate. A variety of immunoassay formats may be used to select antibodies specifically immunoreactive with a particular protein. For example, solid-phase ELISA immunoassays are routinely used to select antibodies specifically immunoreactive with a protein. See, for example, Harlow & Lane, *Antibodies, A Laboratory Manual* (1988), for a description of immunoassay formats and conditions that can be used to determine specific immunoreactivity.

The phrase **"selectively associates with"** refers to the ability of a nucleic acid to "selectively hybridize" with another as defined *supra,* or the ability of an antibody to "selectively (or specifically) bind" to a protein, as defined *supra.*

"**siRNA**" refers to small interfering RNAs, that are capable of causing interference and can cause post-transcriptional silencing of specific genes in cells, for example, mammalian cells (including human cells) and in the body, for example, mammalian bodies (including humans). The phenomenon of RNA interference is described and discussed in Bass, Nature 411: 428-29 (2001); Elbahir et al., Nature 411: 494-98 (2001); and Fire et al., Nature 391: 806-11 (1998), where methods of making interfering RNA also are discussed. The siRNAs based upon the sequence disclosed herein (for example, GenBank accession # NM_025195 for WIP1 mRNA sequence) is less than 100 base pairs ("bps") in length and constituency and preferably is about 30 bps or shorter, and can be made by approaches known in the art, including the use of complementary DNA strands or synthetic approaches. The siRNAs are capable of causing interference and can cause post-transcriptional silencing of specific genes in cells, for example, mammalian cells (including human cells) and in the body, for example, mammalian bodies (including humans). Exemplary siRNAs according to the invention could have up to 29 bps, 25 bps, 22 bps, 21 bps, 20 bps, 15 bps, 10 bps, 5 bps or any integer thereabout or therebetween.

The term **"transgene"** refers to a nucleic acid sequence encoding, for example, one of the WIP1 polypeptides, or an antisense transcript thereto, which is partly or entirely heterologous, i.e., foreign, to the transgenic animal or cell into which it is introduced, or, is homologous to an endogenous gene of the transgenic animal or cell into which it is introduced, but which is designed to be inserted, or is inserted, into the animal's genome in such a way as to alter the genome of the cell into which it is inserted (for example, it is inserted at a location which differs from that of the natural gene or its insertion results in a knockout). A transgene can include one or more transcriptional regulatory sequences and any other nucleic acid, (for example, as intron), that may be necessary for optimal expression of a selected nucleic acid.

A **"transgenic animal"** refers to any animal, preferably a non-human mammal, transgenic and chimeric animals of most vertebrate species. Such species include, but are not limited to, non-human mammals, including rodents, for example, mice and rats, rabbits, bird or an amphibian, ovines, for example, sheep and goats, porcines, for example, pigs, and bovines, for example, cattle and buffalo in which one or more of the cells of the animal contain heterologous nucleic acid introduced by way of human intervention, for example, by transgenic techniques well known in the art. The nucleic acid is introduced into the cell, directly or indirectly by introduction into a precursor of the cell, by way of deliberate genetic manipulation, for example, by microinjection or by infection with a recombinant virus. The term genetic manipulation does not include classical cross-breeding, or sexual fertilization, but rather is directed to the introduction of a recombinant DNA molecule. This molecule may be integrated within a chromosome, or it may be extrachromosomally replicating DNA. In the typical transgenic animals described herein, the transgene causes cells to express a recombinant form of one of the WIP1 proteins, for example, either agonistic or antagonistic forms. However, transgenic animals in which the recombinant WIP1 gene is silent are also contemplated. Moreover, "transgenic animal" also includes those recombinant animals in which gene disruption of one or more WIP1 gene is caused by human intervention, including both recombination and antisense techniques.

Methods of obtaining transgenic animals are described in, for example, Puhler, A., Ed., Genetic Engineering of Animals, VCH Pub., 1993; Murphy and Carter, Eds., Transgenesis Techniques: Principles and Protocols (Methods in Molecular Biology, Vol. 18), 1993; and Pinkert, CA, Ed., Transgenic Animal Technology: A Laboratory Handbook, Academic Press, 1994.

The term **"knockout construct"** refers to a nucleotide sequence that is designed to decrease or suppress expression of a polypeptide encoded by an endogenous gene in one or more cells of a mammal. The nucleotide sequence used as the knockout construct is typically comprised of (1) DNA from some portion of the endogenous gene (one or more exon sequences, intron sequences, and/or promoter sequences) to be suppressed and (2) a marker sequence used to detect the presence of the knockout construct in the cell. The knockout construct can be inserted into a cell containing the endogenous gene to be knocked out. The knockout construct can then integrate with one or both alleles of an endogenous gene, for example, WIP1 gene, and such integration of the knockout construct can prevent or interrupt transcription of the full-length endogenous gene. Integration of the knockout construct into the cellular chromosomal DNA is typically accomplished via homologous recombination (*i.e.,* regions of the knockout construct that are homologous or complementary to endogenous DNA sequences can hybridize to each other when the knockout construct is inserted into the cell; these regions can then recombine so that the knockout construct is incorporated into the corresponding position of the endogenous DNA).

By **"transgenic"** is meant any mammal that includes a nucleic acid sequence, which is inserted into a cell and becomes a part of the genome of the animal that develops from that cell. Such a transgene may be partly or entirely heterologous to the transgenic animal.

Thus, for example, substitution of the naturally occurring WIP 1 gene for a gene from a second species results in an animal that produces the protein of the second species. Substitution of the naturally occurring gene for a gene having a mutation results in an animal that produces the mutated protein. A transgenic mouse expressing the human WIP 1 protein can be generated by direct replacement of the mouse WIP1 subunit with the human gene. These transgenic animals can be critical for drug antagonist studies on animal models for human diseases, and for eventual treatment of disorders or diseases associated with the respective genes. Transgenic mice carrying these mutations will be extremely useful in studying this disease.

A transgenic animal carrying a "**knockout"** of WIP1 gene, would be useful for the establishment of a non-human model for diseases involving such proteins, and to distinguish between the activities of the different WIP1 proteins in an *in vivo* system. **"Knockout mice"** refers to mice whose native or endogenous WIP1 allele or alleles have been disrupted by homologous recombination and which produce no functional WIP1 of their own. Knockout mice may be produced in accordance with techniques known in the art, for example, Thomas, et al., (1999) Immunol. 163:978-84; Kanakaraj, et al. (1998) J. Exp. Med. 187:2073-9; or Yeh, et al., (1997) Immunity 7:715-725.

### WIP1: A Type 2C Protein Phosphatase

The GenBank entry NM_003620 *Homo sapiens* protein phosphatase 1D magnesium-dependent, delta isoform (PPM1D), WIP 1 gene is as shown below:

WIP1 was originally identified as a protein phosphatase gene whose expression is induced in response to gamma or UV radiation in a p53-dependent manner (Fiscella et al., 1997, Proc Natl Acad Sci USA, 94(12): 6048-53). WIP1 is a nuclear protein and a member of the serine/threonine specific protein phosphatase type 2C (PP2C) family. Induction of WIP1 was observed only in cells with an intact p53, suggesting that the WIP1 gene is a downstream target of p53.

The tumor suppressor p53 plays a major role in cellular response to stress; it causes cell cycle arrest and induces apoptosis after DNA damage and certain other cellular stresses (Levine, 1997, Cell, 88, 323-331). It is therefore critical in preserving genomic integrity of many species. In response to DNA damage, p53 protein is transiently stabilized and functionally activated as a transcriptional factor that induces a number of other cellular response genes. See Gu et al, 1997, Cell, 90, 595-606. Phosphorylation at several different serine and threonine residues contributes to stabilization and activation of p53 in this process. See Meek 1998, Int. J. Radiat. Biol. 74, 729-737; Caspari, 2000, Curr. Biol., 10, R315-317. Among several protein kinases that reportedly phosphorylate p53, p38 MARK (mitogen-activated protein kinase) is a prominent p53 activator in response to UV radiation. See Bulavin et al., 1999 EMBL J. 18, 6845-6854; Huang et al., 1999 J Biol. Chem, 274, 12229-12235; and Keller et al., 1999 Biochem. Biophys. Res Commun., 261, 464-471. The activation of stress responsive p38 MARK pathway is one significant event in eukaryotic cells' early response to DNA damage (Kyriakis and Avruch, 1996, J. Biol. Chem., 271, 24313-24316). It represents a perfected cellular protection mechanism that maximizes cellular survival while minimizing carcinogenesis.

Takekawa *et al.* recently showed that WIP1 plays a role in down regulating p38-p53 signaling during the recovery phase of the damaged cell (EMBL J. 2000, 19(23):6517-6526). WIP1 selectively dephosphorylates and inactivates p38 in the cell nucleus. The p38 inhibition by WIP1 attenuates UV induced phosphorylation of p53 which leads to suppression of p53-mediated transcription and apoptosis. WIP1 is also inducible by other stress factors, such as anisomycin, H₂O₂, and methyl methane sulfonate. WIP1 appears, therefore, to exert a negative feedback regulation on p38 MARK-p53 signaling in response to UV radiation.

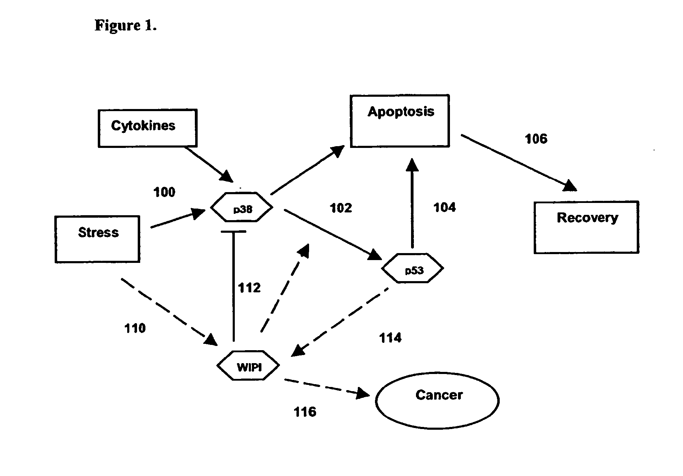

The interactions of WIP 1 and p53-p38 in response to cellular stress stimuli is schematically summarized in Figure 1. Referring to Figure 1, the solid lines 100, 102, 104, and 106 represent the protective mechanism whereby the cells undergo apoptosis in response to stresses and subsequent recover; each step leads to a positive induction or activation of the downstream targets. That is, a stress stimulus such as UV radiation induces p38 (100), which in turn phosphorylates and activates p53 (102); p53 subsequently activates a number of cellular response genes which cause cell cycle arrest and apoptosis (104). At the removal of stress stimulus, however, the cells will recover from the apoptosis state (106). The role of WIP1 is mainly illustrated by the dashed lines 110, 112, 114, and 116 in Figure 1. WIP1 apparently is induced by stress (110), it is a downstream target of p53 which is activated by p53 (114). As discussed above, it negatively feed back on the p38-p53 signaling (112). As a result, it causes cells to deviate from the protective mechanism mediated by the p38-p53 pathways, and thereby become susceptible to cancer development (116). In fact, the present invention shows for the first time that expression of WIP1 can transform normal cells into cells with a more cancerous phenotype. In particular, the present invention shows for the first time that overexpression of WIP1 can suppress cytokine-induced apoptosis in the presence or absence of p53. This insight regarding the p53-independent function of WIP1 means that WIP1 inhibitors should be useful for treating a wide range of tumors regardless of whether the tumor cells express wild-type p53. More details on the possible role of WIP1 in tumorigenesis are discussed in the sections below.

Human chromosome 17q23 is one of the most frequently amplified regions in human breast cancer. More than one gene is located in this region. In a process of characterizing one of the 17q23 amplicons, WIP1 was found amplified and overexpressed in over 15% of human breast tumor samples (see Table 2). Study shown that this amplification is usually associated with aggressive histologic types. Amplification of tumor-promoting gene(s) located on 17q23 may play an important role in the development and/or progression of a substantial proportion of primary breast cancers, particularly those of the invasive histology.

WIP1 was found by DNA microarray analysis of human breast tumor for DNA amplification using the methods described elsewhere. See, for example, US 6,232,068; Pollack et al., Nat. Genet. 23(1):41-46, 1999. Further analysis provided evidence that WIP1 is at the epicenter of amplification region.

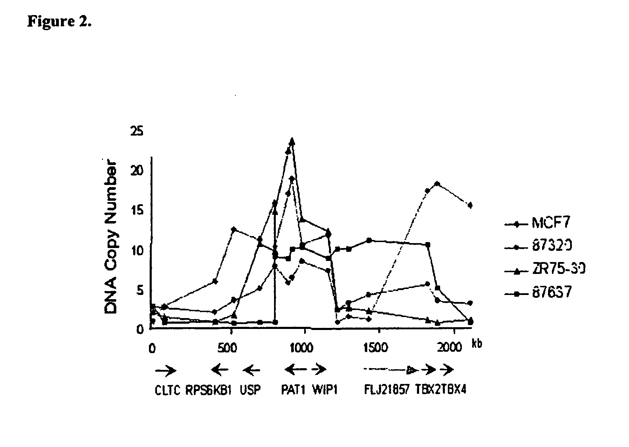

The indicated cell lines or primary tumors were examined for DNA copy number of nearby genes and DNA sequences that map to the boundaries of the amplified regions. TaqMan epicenter data for WIP 1 is shown in Figure 2.

The corresponding genomic DNA sequence from the human genome project was analyzed for the presence of genes. WIP1 was found at the epicenter. Overall WIP1 was found amplified in over 15% of human breast tumors (with 2.5-fold cutoff).

Quantitative RT-PCR analysis with Taqman probes showed that WIP1 was overexpressed in 8/11 (>73%) breast cell line and 3/20 (15%) in primary breast tumor samples. Further, amplification and overexpression have a good correlation (see Tables I and 2).

**Table 1. Amplification and overexpression of WIP1.**

| CELL LINE | AMPLIFICATION OF WIP1 | EXPRESSION OF WIP1 |
|---|---|---|
| BT474 | 3.3 | 18.6 |
| MDAMB361 | 4.2 | 18.7 |
| ZR75-30 | 10.9 | 27.1 |
| MCF7 | 15.4 | 65.9 |
| ZR75-1 | 2 | 5.4 |
| MDAMB 134 | 0.8 | 6.7 |
| MDAMB453 | 1.2 | 9.9 |
| MDAMB157 | 1.1 | 4.4 |
| MDAMB 175 | 1.1 | 4.1 |
| MDAMB330 | 1.2 | 19.1 |
| MDAMB231 | 0.7 | 1.5 |

**Table 2. Amplification and overexpression frequency of WIP 1 in primary tumor samples.**

| | Breast | Colon | Lung | Primary prostate | Metastatic prostate | Ovary |
|---|---|---|---|---|---|---|
| Amplifciation¹ | 16% (27/164) (14x)³ | 0% (0/17) | 3% (1/31) (3.1x) | 0% (0/18) | 0% (0/15) | 0% (0/38) |
| Overexpression² | 15% (3/20) (5x) | 8% (2/25) (6x) | 23% (5/22) (6x) | 0%(0/18) | 47% (7/15) (>100x) | 9% (1/11) (40x) |

| | | | | | | |
|---|---|---|---|---|---|---|
| ¹DNA copy number cutoff for amplification: 2.5X; ²NA overexpression cutoff: 3X; ³The highest observed amplification and overexpression | | | | | | |

The folds of amplification and folds of overexpression were measured by Taqman and RT-Taqman respectively using WIP1 specific fluorogenic Taqman probes. There is a good correlation between and amplification and overexpression (see Tables 1 and 2).

More details on the possible role of WIP1 in tumorigenesis are discussed in the sections below.

### Amplification of WIP1 Gene in Tumors:

The presence of a target gene that has undergone amplification in tumors is evaluated by determining the copy number of the target genes, i.e., the number of DNA sequences in a cell encoding the target protein. Generally, a normal cell has two copies of a given autosomal gene. The copy number can be increased, however, by gene amplification or duplication, for example, in cancer cells, or reduced by deletion. Methods of evaluating the copy number of a particular gene are well known in the art, and include, *inter alia,* hybridization and amplification based assays.

Any of a number of hybridization based assays can be used to detect the copy number of the WIP 1 gene in the cells of a biological sample. One such method is Southern blot (see Ausubel *et al.,* or Sambrook *et al., supra),* where the genomic DNA is typically fragmented, separated electrophoretically, transferred to a membrane, and subsequently hybridized to a WIP1 specific probe. Comparison of the intensity of the hybridization signal from the probe for the target region with a signal from a control probe from a region of normal nonamplified, single-copied genomic DNA in the same genome provides an estimate of the relative WIP1 copy number, corresponding to the specific probe used. An increased signal compared to control represents the presence of amplification.

A methodology for determining the copy number of the WIP 1 gene in a sample is in situ hybridization, for example, fluorescence in situ hybridization (FISH) (see Angerer, 1987 *Meth. Enzymol* 152: 649). Generally, in situ hybridization comprises the following major steps: (1) fixation of tissue or biological structure to be analyzed; (2) prehybridization treatment of the biological structure to increase accessibility of target DNA, and to reduce nonspecific binding; (3) hybridization of the mixture of nucleic acids to the nucleic acid in the biological structure or tissue; (4) post-hybridization washes to remove nucleic acid fragments not bound in the hybridization, and (5) detection of the hybridized nucleic acid fragments. The probes used in such applications are typically labeled, for example, with radioisotopes or fluorescent reporters. Preferred probes are sufficiently long, for example, from about 50, 100, or 200 nucleotides to about 1000 or more nucleotides, to enable specific hybridization with the target nucleic acid(s) under stringent conditions.

Another alternative methodology for determining number of DNA copies is comparative genomic hybridization (CGH). In comparative genomic hybridization methods, a "test" collection of nucleic acids is labeled with a first label, while a second collection (for example, from a normal cell or tissue) is labeled with a second label. The ratio of hybridization of the nucleic acids is determined by the ratio of the first and second labels binding to each fiber in an array. Differences in the ratio of the signals from the two labels, for example, due to gene amplification in the test collection, is detected and the ratio provides a measure of the WIP1 gene copy number, corresponding to the specific probe used. A cytogenetic representation of DNA copy-number variation can be generated by CGH, which provides fluorescence ratios along the length of chromosomes from differentially labeled test and reference genomic DNAs.

Hybridization protocols suitable for use with the methods of the invention are described, for example, in Albertson (1984) EMBO J. 3:1227-1234; Pinkel (1988) Proc. Natl. Acad. Sci. USA 85:9138-9142; EPO Pub. No. 430:402; Methods in Molecular Biology, Vol. 33*: In Situ* Hybridization Protocols, Choo, ed., Humana Press, Totowa, NJ (1994).

Amplification-based assays also can be used to measure the copy number of the WIP1 gene. In such assays, the corresponding WIP1 nucleic acid sequences act as a template in an amplification reaction (for example, Polymerase Chain Reaction or PCR). In a quantitative amplification, the amount of amplification product will be proportional to the amount of template in the original sample. Comparison to appropriate controls provides a measure of the copy number of the WIP1 gene, corresponding to the specific probe used, according to the principle discussed above. Methods of real-time quantitative PCR using Taqman probes are well known to in the art. Detailed protocols for real-time quantitative PCR are provided, for example, for RNA in: Gibson et al., 1996, A novel method for real time quantitative RT-PCR. Genome Res. 10:995-1001; and for DNA in: Heid et al., 1996, Real time quantitative PCR. Genome Res. 10:986-994.

A TaqMan-based assay can also be used to quantify WIP1 polynucleotides. TaqMan based assays use a fluorogenic oligonucleotide probe that contains a 5' fluorescent dye and a 3' quenching agent. The probe hybridizes to a PCR product, but cannot itself be extended due to a blocking agent at the 3' end. When the PCR product is amplified in subsequent cycles, the 5' nuclease activity of the polymerase, for example, AmpliTaq, results in the cleavage of the TaqMan probe. This cleavage separates the 5' fluorescent dye and the 3' quenching agent, thereby resulting in an increase in fluorescence as a function of amplification (see, for example, http://www2.perkin-elmer.com).

Other suitable amplification methods include, but are not limited to, ligase chain reaction (LCR) (see, Wu and Wallace, 1989, Genomics 4: 560; Landegren et al., 1988 Science 241: 1077; and Barringer et al., 1990, Gene 89: 117), transcription amplification (Kwoh et al., 1989, Proc. Natl. Acad. Sci. USA 86: 1173), self-sustained sequence replication (Guatelli et al., 1990, Proc. Nat. Acad. Sci. USA 87: 1874), dot PCR, and linker adapter PCR, *etc.*

One powerful method for determining DNA copy numbers uses microarray-based platforms. Microarray technology may be used because it offers high resolution. For example, the traditional CGH generally has a 20 Mb limited mapping resolution; whereas in microarray-based CGH, the fluorescence ratios of the differentially labeled test and reference genomic DNAs provide a locus-by-locus measure of DNA copy-number variation, thereby achieving increased mapping resolution. Details of a microarray method can be found in the literature. See, for example, US 6,232,068; Pollack et al., Nat Genet, 1999, 23(1):41-6.

As demonstrated in the Examples set forth herein, the WIP1 gene is frequently amplified in certain cancers, particularly breast cancers; and it resides at the epicenter of the amplified chromosome region. All samples showing WIP1 gene amplification in Table 2 also demonstrate overexpression of WIP1 mRNA. The WIP1 gene has these characteristic features of overexpression, amplification, and the correlation between the two, and these features are shared with other well studied oncogenes (Yoshimoto et al., 1986, JPN J Cancer Res, 77(6):540-5; Knuutila et al., Am J Pathol 1998 152(5):1107-23). The WIP1 genes are accordingly used in the present invention as a target for cancer diagnosis and treatment.

### Frequent Overexpression of WIP1 Gene in Cancers:

The expression levels of the WIP1 gene in a variety of tumors were examined. As demonstrated in the examples *infra,* WIP1 gene is overexpressed in breast, lung, colon, ovarian, and prostate cancer cell lines. Detection and quantification of the WIP1 gene expression may be carried out through direct hybridization based assays or amplification based assays. The hybridization based techniques for measuring gene transcript are known to those skilled in the art (Sambrook et al., 1989. Molecular Cloning: A Laboratory Manual, 2d Ed. vol. 1-3, Cold Spring Harbor Press, NY). For example, one method for evaluating the presence, absence, or quantity of the WIP 1 gene is by Northern blot. Isolated mRNAs from a given biological sample are electrophoresed to separate the mRNA species, and transferred from the gel to a membrane, for example, a nitrocellulose or nylon filter. Labeled WIP1 probes are then hybridized to the membrane to identify and quantify the respective mRNAs. The example of amplification based assays include RT-PCR, which is well known in the art (Ausubel *et al., Current Protocols in Molecular Biology,* eds. 1995 supplement). Quantitative RT-PCR is used preferably to allow the numerical comparison of the level of respective WIP1 mRNAs in different samples.

### Cancer Diagnosis and Therapies Using WIP1:

### Detection and Measurement of the WIP1 Gene and Protein:

### A. Overexpression and Amplification of the WIP1 Gene:

The WIP 1 gene and its expressed gene product can be used for diagnosis, prognosis, rational drug design, and other therapeutic intervention of tumors and cancers (for example, breast cancer, lung cancer, prostate cancer, ovarian cancer, colon cancer, *etc.*).

Detection and measurement of amplification and/or overexpression of the WIP 1 gene in a biological sample taken from a patient indicates that the patient may have developed a tumor. Particularly, the presence of amplified WIP 1 DNA leads to a diagnosis of cancer, for example, breast cancer, lung cancer, prostate cancer, ovarian cancer, or colon cancer with high probability of accuracy. The present invention therefore provides, in one aspect, methods for diagnosing a cancer or tumor in a mammalian tissue by measuring the levels of WIP1 mRNA expression in samples taken from the tissue of suspicion, and determining whether WIP1 is overexpressed in the tissue. The various techniques, including hybridization based and amplification based methods, for measuring and evaluating mRNA levels are provided herein as discussed *supra.* The present invention also provides, in another aspect, methods for diagnosing a cancer or tumor in a mammalian tissue by measuring the numbers of WIP1 DNA copy in samples taken from the tissue of suspicion, and determining whether the WIP1 gene is amplified in the tissue. The various techniques, including hybridization based and amplification based methods, for measuring and evaluating DNA copy numbers are provided herein as discussed *supra.* The present invention thus provides methods for detecting amplified genes at DNA level and increased expression at RNA level, wherein both the results are indicative of tumor progression.

### B. Detection of the WIP1 Protein:

According to the present invention, the detection of increased WIP1 protein level in a biological subject may also suggest the presence of a precancerous or cancerous condition in the tissue source of the sample. Protein detection for tumor and cancer diagnostics and prognostics can be carried out by immunoassays, for example, using antibodies directed against a target gene, for example, WIP 1. Any methods that are known in the art for protein detection and quantitation can be used in the methods of this invention, including, *inter alia,* electrophoresis, capillary electrophoresis, high performance liquid chromatography (HPLC), thin layer chromatography (TLC), hyperdiffusion chromatography, immunoelectrophoresis, radioimmunoassay (RIA), enzyme-linked immunosorbent assays (ELISAs), immuno-flouorescent assays, Western Blot, *etc.* Protein from the tissue or cell type to be analyzed may be isolated using standard techniques, for example, as described in Harlow and Lane, Antibodies: A Laboratory Manual (Cold Spring Harbor Laboratory Press, Cold Spring Harbor, N.Y. 1988).

The antibodies (or fragments thereof) useful in the present invention can, additionally, be employed histologically, as in immunofluorescence or immunoelectron microscopy, for in situ detection of target gene peptides. In situ detection can be accomplished by removing a histological specimen from a patient, and applying thereto a labeled antibody of the present invention. The antibody (or its fragment) is preferably applied by overlaying the labeled antibody (or fragment) onto a biological sample. Through the use of such a procedure, it is possible to determine not only the presence of the target gene product, for example, WIP1 protein, but also their distribution in the examined tissue. Using the present invention, a skilled artisan will readily perceive that any of a wide variety of histological methods (for example, staining procedures) can be modified to achieve such in situ detection.

The biological sample that is subjected to protein detection can be brought in contact with and immobilized on a solid phase support or carrier, for example, nitrocellulose, or other solid support which is capable of immobilizing cells, cell particles, or soluble proteins. The support can then be washed with suitable buffers followed by treatment with the detectably labeled fingerprint gene specific antibody. The solid phase support can then be washed with the buffer a second time to remove unbound antibody. The amount of bound label on the solid support can then be detected by conventional means.

A target gene product-specific antibody, for example, a WIP1 antibody can be detectably labeled, in one aspect, by linking the same to an enzyme, for example, horseradish peroxidase, alkaline phosphatase, or glucoamylase, and using it in an enzyme immunoassay (EIA) (see, for example, Voller, A., 1978, The Enzyme Linked Immunosorbent Assay (ELISA), Diagnostic Horizons, 2:1-7; Voller et al., 1978, J. Clin. Pathol., 31:507-520; Butler, J. E., 1981, Meth. Enzymol., 73:482-523; Maggio, E. (ed.), 1980, Enzyme Immunoassay, CRC Press, Boca Raton, Fla.; and Ishikawa et al. (eds), 1981, Enzyme Immunoassay, Kgaku Shoin, Tokyo.) The enzyme bound to the antibody reacts with an appropriate substrate, preferably a chromogenic substrate, in such a manner as to produce a chemical moiety that can be detected, for example, by spectrophotometric or fluorimetric means, or by visual inspection.

In a related aspect, therefore, the present invention provides the use of WIP1 antibodies in cancer diagnosis and intervention. Antibodies that specifically bind to WIP 1 protein and polypeptides can be produced by a variety of methods. Such antibodies may include, but are not limited to, polyclonal antibodies, monoclonal antibodies (mAbs), humanized or chimeric antibodies, single chain antibodies, Fab fragments, F(ab')₂ fragments, fragments produced by a Fab expression library, anti-idiotypic (anti-Id) antibodies, and epitope-binding fragments of any of the above.

Such antibodies can be used, for example, in the detection of the target gene, WIP1, or its fingerprint or pathway genes involved in a particular biological pathway, which may be of physiological or pathological importance. These potential pathways or fingerprint genes, for example, may interact with protein phosphatase activity of WIP1 and be involved in tumorigenesis. The WIP1 antibodies can also be used in a method for the inhibition of W1P1 activity, respectively. Thus, such antibodies can be used in treating tumors and cancers (for example, breast cancer, lung cancer, prostate cancer, ovarian cancer, or colon cancer); they may also be used in diagnostic procedures whereby patients are tested for abnormal levels of WIP1 protein, and/or fingerprint or pathway gene protein associated with WIP1, and for the presence of abnormal forms of such protein.

To produce antibodies to WIP1 protein, a host animal is immunized with the protein, or a portion thereof. Such host animals can include, but are not limited to, rabbits, mice, and rats. Various adjuvants can be used to increase the immunological response, depending on the host species, including but not limited to Freund's (complete and incomplete), mineral gels, for example, aluminum hydroxide, surface active substances, for example, lysolecithin, pluronic polyols, polyanions, peptides, oil emulsions, keyhole limpet hemocyanin (KLH), dinitrophenol (DNP), and potentially useful human adjuvants, for example, BCG (*Bacille Calmette-Guerin*) and *Corynebacterium parvum.*

Monoclonal antibodies, which are homogeneous populations of antibodies to a particular antigen, for example, WIP1 as in the present invention, can be obtained by any technique which provides for the production of antibody molecules by continuous cell lines in culture. These include, but are not limited to the hybridoma technique of Kohler and Milstein, (Nature, 256:495-497, 1975; and U.S. Pat. No. 4,376,110), the human B-cell hybridoma technique (Kosbor et al., Immunology Today, 4:72, 1983; Cole et al., Proc. Natl. Acad Sci. U.S.A., 80:2026-2030, 1983), and the BV-hybridoma technique (Cole et al., Monoclonal Antibodies And Cancer Therapy (Alan R. Liss, Inc. 1985), pp. 77-96. Such antibodies can be of any immunoglobulin class including IgG, IgM, IgE, IgA, IgD and any subclass thereof. The hybridoma producing the mAb of this invention can be cultivated *in vitro* or *in vivo.* Production of high titers of mAbs *in vivo* makes this the presently preferred method of production.

In addition, techniques developed for the production of "chimeric antibodies" can be made by splicing the genes from a mouse antibody molecule of appropriate antigen specificity together with genes from a human antibody molecule of appropriate biological activity (see, Morrison et al., Proc. Natl. Acad. Sci. USA, 81:6851-6855, 1984; Neuberger et al., Nature, 312:604-608, 1984; Takeda et al., Nature, 314:452-454, 1985; and U.S. Pat. No. 4,816,567). A chimeric antibody is a molecule in which different portions are derived from different animal species, for example, those having a variable region derived from a murine mAb and a container region derived from human immunoglobulin.

Alternatively, techniques described for the production of single chain antibodies (for example, U.S. Pat. No. 4,946,778; Bird, Science, 242:423-426, 1988; Huston et al., Proc. Natl. Acad. Sci. U.S.A., 85:5879-5883, 1988; and Ward et al., Nature, 334:544-546, 1989), and for making humanized monoclonal antibodies (U.S. Pat. No. 5,225,539), can be used to produce anti-differentially expressed or anti-pathway gene product antibodies.

Antibody fragments that recognize specific epitopes can be generated by known techniques. For example, such fragments include but are not limited to: the F(ab')₂ fragments that can be produced by pepsin digestion of the antibody molecule, and the Fab fragments that can be generated by reducing the disulfide bridges of the F(ab')₂ fragments. Alternatively, Fab expression libraries can be constructed (Huse et al., Science, 246:1275-1281, 1989) to allow rapid and easy identification of monoclonal Fab fragments with the desired specificity.

### C. Use of WIP1 Modulators in Cancer Diagnostics:

Aside from antibodies, the present invention provides, in another aspect, the diagnostic and therapeutic utilities of other molecules and compounds that interact with WIP1 protein. Specifically, such compounds can include, but are not limited to, proteins or peptides, for example, soluble peptides, for example, Ig-tailed fusion peptides, comprising extracellular portions of transmembrane proteins of the target, if they exist, and members of random peptide libraries (see, for example, Lam et al., Nature, 354:82-84, 1991; Houghton et al., Nature, 354:84-86, 1991), made of D- and/or L-configuration amino acids, phosphopeptides (including, but not limited to, members of random or partially degenerate phosphopeptide libraries; see, for example, Songyang et al., Cell, 72:767-778, 1993), and small organic or inorganic molecules. In this aspect, the present invention provides a number of methods and procedures to assay or identify compounds that bind to target, *i.e.,* WIP1 protein, or to any cellular protein that may interact with the target, and compounds that may interfere with the interaction of the target with other cellular proteins.

*In vitro* assay systems are provided that are capable of identifying compounds that specifically bind to the target gene product, for example, WIP1 protein. The assays all involve the preparation of a reaction mixture of the target gene product, for example, WIP1 protein and a test compound under conditions and for a time sufficient to allow the two components to interact and bind, thus forming a complex that can be removed and/or detected in the reaction mixture. These assays can be conducted in a variety of ways. For example, one method involves anchoring the target protein or the test substance to a solid phase, and detecting target protein - test compound complexes anchored to the solid phase at the end of the reaction. In one aspect of such a method, the target protein can be anchored onto a solid surface, and the test compound, which is not anchored, can be labeled, either directly or indirectly. In practice, microtiter plates can be used as the solid phase. The anchored component can be immobilized by non-covalent or covalent attachments. Non-covalent attachment can be accomplished by simply coating the solid surface with a solution of the protein and drying. Alternatively, an immobilized antibody, preferably a monoclonal antibody, specific for the protein to be immobilized can be used to anchor the protein to the solid surface. The surfaces can be prepared in advance and stored.

To conduct the assay, the non-immobilized component is added to the coated surface containing the anchored component. After the reaction is complete, unreacted components are removed, for example, by washing, and complexes anchored on the solid surface are detected. Where the previously immobilized component is pre-labeled, the detection of label immobilized on the surface indicates that complexes were formed. Where the previously non-immobilized component is not pre-labeled, an indirect label can be used to detect complexes anchored on the surface; for example, using a labeled antibody specific for the immobilized component (the antibody, in turn, can be directly labeled or indirectly labeled with a labeled anti-Ig antibody). Alternatively, the reaction can be conducted in a liquid phase, the reaction products separated from unreacted components, and complexes detected, for example, using an immobilized antibody specific for a target gene or the test compound to anchor any complexes formed in solution, and a labeled antibody specific for the other component of the possible complex to detect anchored complexes.

Assays are also provided for identifying any cellular protein that may interact with the target protein, *i.e*., WIP1 protein. Any method suitable for detecting protein-protein interactions can be used to identify novel interactions between target protein and cellular or extracellular proteins. Those cellular or extracellular proteins may be involved in certain cancers, for example, breast cancer, lung cancer, prostate cancer, ovarian cancer, or colon cancer, and represent certain tumorigenic pathways including the target, for example, WIP 1. They may thus be denoted as pathway genes.

Methods, for example, co-immunoprecipitation and co-purification through gradients or chromatographic columns, can be used to identify protein-protein interactions engaged by the target protein. The amino acid sequence of the target protein, *i.e.,* WIP1 protein or a portion thereof (see SWISS-PROT record 015297), is useful in identifying the pathway gene products or other proteins that interact with WIP1 protein. The amino acid sequence can be derived from the nucleotide sequence, or from published database records (SWISS-PROT, PIR, EMBL); it can also be ascertained using techniques well known to a skilled artisan, for example, the Edman degradation technique (see, for example, Creighton, *Proteins: Structures and Molecular Principles,* 1983, W. H. Freeman & Co., N.Y., 34-49). The nucleotide subsequences of the target gene, for example, WIP1, can be used in a reaction mixture to screen for pathway gene sequences. Screening can be accomplished, for example, by standard hybridization or PCR techniques. Techniques for the generation of oligonucleotide mixtures and the screening are well known (see, for example, Ausubel, *supra,* and Innis et al. (eds.), PCR Protocols: A Guide to Methods and Applications, 1990, Academic Press, Inc., New York).

By way of example, the yeast two-hybrid system which is often used in detecting protein interactions *in vivo* is discussed herein. Chien *et al.* has reported the use of a version of the yeast two-hybrid system *(*Proc. Natl. Acad Sci. USA, 1991, 88:9578-9582); it is commercially available from Clontech (Palo Alto, Calif.). Briefly, utilizing such a system, plasmids are constructed that encode two hybrid proteins: the first hybrid protein comprises the DNA-binding domain of a transcription factor, for example, activation protein, fused to a known protein, in this case, a protein known to be involved in a tumor or cancer, and the second hybrid protein comprises the transcription factor's activation domain fused to an unknown protein that is encoded by a cDNA which has been recombined into this plasmid as part of a cDNA library. The plasmids are transformed into a strain of the yeast *Saccharomyces cerevisiae* that contains a reporter gene, for example, *lacZ,* whose expression is regulated by the transcription factor's binding site. Either hybrid protein alone cannot activate transcription of the reporter gene. The DNA binding hybrid protein cannot activate transcription because it does not provide the activation domain function, and the activation domain hybrid protein cannot activate transcription because it lacks the domain required for binding to its target site, i.e., it cannot localize to the transcription activator protein's binding site. Interaction between the DNA binding hybrid protein and the library encoded protein reconstitutes the functional transcription factor and results in expression of the reporter gene, which is detected by an assay for the reporter gene product.

The two-hybrid system or similar methods can be used to screen activation domain libraries for proteins that interact with a known "bait" gene product. The WIP1 gene product, involved in a number of tumors and cancers, is such a bait according to the present invention. Total genomic or cDNA sequences are fused to the DNA encoding an activation domain. This library and a plasmid encoding a hybrid of the bait gene product, *i.e.,* WIP1 protein or polypeptides, fused to the DNA-binding domain are co-transformed into a yeast reporter strain, and the resulting transformants are screened for those that express the reporter gene. For example, the bait gene WIP1 can be cloned into a vector such that it is translationally fused to the DNA encoding the DNA-binding domain of the GAL4 protein. The colonies are purified and the (library) plasmids responsible for reporter gene expression are isolated. The inserts in the plasmids are sequenced to identify the proteins encoded by the cDNA or genomic DNA.

A cDNA library of a cell or tissue source that expresses proteins predicted to interact with the bait gene product, for example, WIP1, can be made using methods routinely practiced in the art. According to the particular system described herein, the library is generated by inserting the cDNA fragments into a vector such that they are translationally fused to the activation domain of GAL4. This library can be cotransformed along with the bait gene-GAL4 fusion plasmid into a yeast strain which contains a *lacZ* gene whose expression is controlled by a promoter which contains a GAL4 activation sequence. A cDNA encoded protein, fused to GAL4 activation domain, that interacts with the bait gene product will reconstitute an active GAL4 transcription factor and thereby drive expression of the *lacZ* gene. Colonies that express *lacZ* can be detected by their blue color in the presence of X-gal. cDNA containing plasmids from such a blue colony can then be purified and used to produce and isolate the WIP1-interacting protein using techniques routinely practiced in the art.

In another aspect, the present invention also provides assays for compounds that interfere with gene and cellular protein interactions involving the target WIP1. The target gene product, for example, WIP 1 protein, may interact *in vivo* with one or more cellular or extracellular macromolecules, for example, proteins and nucleic acid molecules. Such cellular and extracellular macromolecules are referred to as "binding partners." Compounds that disrupt such interactions can be used to regulate the activity of the target gene product, for example, WIP I protein, especially mutant target gene product. Such compounds can include, but are not limited to, molecules, for example, antibodies, peptides and other chemical compounds.

The assay systems all involve the preparation of a reaction mixture containing the target gene product WIP1 protein, and the binding partner under conditions and for a time sufficient to allow the two products to interact and bind, thus forming a complex. To test a compound for inhibitory activity, the reaction mixture is prepared in the presence and absence of the test compound. The test compound can be initially included in the reaction mixture, or can be added at a time subsequent to the addition of a target gene product and its cellular or extracellular binding partner. Control reaction mixtures are incubated without the test compound or with a placebo. The formation of complexes between the target gene product WIP1 protein and the cellular or extracellular binding partner is then detected. The formation of a complex in the control reaction, but not in the reaction mixture containing the test compound, indicates that the compound interferes with the interaction of the target gene product WIP1 protein and the interactive binding partner. Additionally, complex formation within reaction mixtures containing the test compound and normal target gene product can be compared to complex formation within reaction mixtures containing the test compound and mutant target gene product. This comparison can be important in the situation where it is desirable to identify compounds that disrupt interactions of mutant but not normal target gene product.

The assays can be conducted in a heterogeneous or homogeneous format. Heterogeneous assays involve anchoring either the target gene product WIP I protein or the binding partner to a solid phase and detecting complexes anchored to the solid phase at the end of the reaction, as described above. In homogeneous assays, the entire reaction is carried out in a liquid phase, as described below. In either approach, the order of addition of reactants can be varied to obtain different information about the compounds being tested. For example, test compounds that interfere with the interaction between the target gene product WIP 1 protein and the binding partners, for example, by competition, can be identified by conducting the reaction in the presence of the test substance; i.e., by adding the test substance to the reaction mixture prior to or simultaneously with the target gene product WIP1 protein and interactive cellular or extracellular binding partner. Alternatively, test compounds that disrupt preformed complexes, for example, compounds with higher binding constants that displace one of the components from the complex, can be tested by adding the test compound to the reaction mixture after complexes have been formed.

In a homogeneous assay, a preformed complex of the target gene product and the interactive cellular or extracellular binding partner product is prepared in which either the target gene products or their binding partners are labeled, but the signal generated by the label is quenched due to complex formation (see, for example, Rubenstein, U.S. Pat. No. 4,109,496). The addition of a test substance that competes with and displaces one of the species from the preformed complex will result in the generation of a signal above background. The test substances that disrupt the interaction between the target gene product WIP1 protein and cellular or extracellular binding partners can thus be identified.

In one aspect, the target gene product WIP 1 protein can be prepared for immobilization using recombinant DNA techniques. For example, the target WIP1 coding region can be fused to a glutathione-S-transferase (GST) gene using a fusion vector, for example, pGEX-5X-1, in such a manner that its binding activity is maintained in the resulting fusion product. The interactive cellular or extracellular binding partner product is purified and used to raise a monoclonal antibody, using methods routinely practiced in the art. This antibody can be labeled with the radioactive isotope ¹²⁵I, for example, by methods routinely practiced in the art.

In a heterogeneous assay, the GST-Target gene fusion product is anchored, for example, to glutathione-agarose beads. The interactive cellular or extracellular binding partner is then added in the presence or absence of the test compound in a manner that allows interaction and binding to occur. At the end of the reaction period, unbound material is washed away, and the labeled monoclonal antibody can be added to the system and allowed to bind to the complexed components. The interaction between the target gene product WIP1 protein and the interactive cellular or extracellular binding partner is detected by measuring the corresponding amount of radioactivity that remains associated with the glutathione-agarose beads. A successful inhibition of the interaction by the test compound will result in a decrease in measured radioactivity. Alternatively, the GST-target gene fusion product and the interactive cellular or extracellular binding partner can be mixed together in liquid in the absence of the solid glutathione-agarose beads. The test compound is added either during or after the binding partners are allowed to interact. This mixture is then added to the glutathione-agarose beads and unbound material is washed away. Again, the extent of inhibition of the binding partner interaction can be detected by adding the labeled antibody and measuring the radioactivity associated with the beads.

In other aspects of the invention, these same techniques are employed using peptide fragments that correspond to the binding domains of the target gene product, for example, WIP1 protein and the interactive cellular or extracellular binding partner (where the binding partner is a product), in place of one or both of the full-length products. Any number of methods routinely practiced in the art can be used to identify and isolate the protein's binding site. These methods include, but are not limited to, mutagenesis of one of the genes encoding one of the products and screening for disruption of binding in a co-immunoprecipitation assay.

Additionally, compensating mutations in the gene encoding the second species in the complex can be selected. Sequence analysis of the genes encoding the respective products will reveal mutations that correspond to the region of the product involved in interactive binding. Alternatively, one product can be anchored to a solid surface using methods described above, and allowed to interact with and bind to its labeled binding partner, which has been treated with a proteolytic enzyme, for example, trypsin. After washing, a short, labeled peptide comprising the binding domain can remain associated with the solid material, which can be isolated and identified by amino acid sequencing. Also, once the gene coding for the cellular or extracellular binding partner product is obtained, short gene segments can be engineered to express peptide fragments of the product, which can then be tested for binding activity and purified or synthesized.

### D. Methods for Cancer Treatment Using WIP1 Modulator:

In another aspect, the present invention provides methods for treating or controlling a cancer or tumor and the symptoms associated therewith. Any of the binding compounds, for example, those identified in the aforementioned assay systems, can be tested for the ability to prevent and/or ameliorate symptoms of tumors and cancers (for example, breast cancer, lung cancer, colon cancer, ovarian cancer, or prostate cancer). As used herein, inhibit, control, ameliorate, prevent, treat, and suppress collectively and interchangeably mean stopping or slowing cancer formation, development, or growth and eliminating or reducing cancer symptoms. Cell-based and animal model-based trial systems for evaluating the ability of the tested compounds to prevent and/or ameliorate tumors and cancers symptoms are used according to the present invention.

For example, cell based systems can be exposed to a compound suspected of ameliorating breast tumor or cancer symptoms, at a sufficient concentration and for a time sufficient to elicit such an amelioration in the exposed cells. After exposure, the cells are examined to determine whether one or more tumor or cancer phenotypes has been altered to resemble a more normal or more wild-type, non-cancerous, phenotype. Further, the levels of WIP1 mRNA expression and DNA amplification within these cells may be determined, according to the methods provided *supra.* A decrease in the observed level of expression and amplification would indicate to a certain extent the successful intervention of tumors and cancers (for example, breast cancer, lung cancer, colon cancer, ovarian cancer, or prostate cancer).

In addition, animal models can be used to identify compounds for use as drugs and pharmaceuticals that are capable of treating or suppressing symptoms of tumors and cancers. For example, animal models can be exposed to a test compound at a sufficient concentration and for a time sufficient to elicit such an amelioration in the exposed animals. The response of the animals to the exposure can be monitored by assessing the reversal of symptoms associated with the tumor or cancer, or by evaluating the changes in DNA copy number and levels of mRNA expression of the target gene, for example, WIP1. Any treatments which reverse any symptom of tumors and cancers, and/or which reduce overexpression and amplification of the target WIP1 gene may be considered as candidates for therapy in humans. Dosages of test agents can be determined by deriving dose-response curves.

Moreover, fingerprint patterns or gene, protein expression profiles can be characterized for known cell states, for example, normal or known pre-neoplastic, neoplastic, or metastatic states, within the cell- and/or animal-based model systems. Subsequently, these known fingerprint patterns can be compared to ascertain the ability of a test compound to modify such fingerprint patterns, and to cause the pattern to more closely resemble that of a normal fingerprint pattern. For example, administration of a compound which interacts with and affects WIP1 gene expression and amplification may cause the fingerprint pattern of a precancerous or cancerous model system to more closely resemble a control, normal system; such a compound thus will have therapeutic utilities in treating the cancer. In other situations, administration of a compound may cause the fingerprint pattern of a control system to begin to mimic tumors and cancers (for example, breast cancer, lung cancer, prostate cancer, ovarian cancer, or colon cancer); such a compound therefore acts as a tumorigenic agent, which in turn can serve as a target for therapeutic interventions of the cancer and its diagnosis.

### E. Methods for Monitoring Efficacy of Cancer Treatment:

In a further aspect, the present invention provides methods for monitoring the efficacy of a therapeutic treatment regimen of cancer and methods for monitoring the efficacy of a compound in clinical trials for inhibition of tumors. The monitoring can be accomplished by detecting and measuring, in the biological samples taken from a patient at various time points during the course of the application of a treatment regimen for treating a cancer or a clinical trial, the changed levels of expression or amplification of the target gene, for example, WIP1. A level of expression and/or amplification that is lower in samples taken at the later time of the treatment or trial then those at the earlier date indicates that the treatment regimen is effective to control the cancer in the patient, or the compound is effective in inhibiting the tumor. The time course studies should be so designed that sufficient time is allowed for the treatment regimen or the compound to exert its effect.

Therefore, the influence of compounds on tumors and cancers can be monitored both in a clinical trial and in a basic drug screening. In a clinical trial, for example, tumor cells can be isolated from breast tumors removed by surgery, and RNA prepared and analyzed by Northern blot analysis or TaqMan RT-PCR as described herein, or alternatively by measuring the amount of protein produced. The fingerprint expression profiles thus generated can serve as putative biomarkers for breast or lung tumors or cancers. Particularly, the expression of WIP 1 serves as one such biomarker. Thus, by monitoring the level of expression of the differentially or over-expressed genes, for example, WIP1, an effective treatment protocol can be developed using suitable chemotherapeutic anticancer drugs.

### F. Use of Modulators to WIP1 Nucleotides in Cancer Treatment:

In another further aspect of this invention, additional compounds and methods for treatment of tumors are provided. Symptoms of tumors and cancers can be controlled by, for example, target gene modulation, and/or by a depletion of the precancerous or cancerous cells. Target gene modulation can be of a negative or positive nature, depending on whether the target resembles a gene (for example, tumorigenic) or a tumor suppressor gene (for example, tumor suppressive). That is, inhibition, *i.e.,* a negative modulation, of an oncogene-like target gene or stimulation, *i.e.,* a positive modulation, of a tumor suppressor-like target gene will control or ameliorate the tumor or cancer in which the target gene is involved. More precisely, "negative modulation" refers to a reduction in the level and/or activity of target gene or its product, for example, WIP1, relative to the level and/or activity of the target gene product in the absence of the modulatory treatment. "Positive modulation" refers to an increase in the level and/or activity of target gene product, for example, WIP1, relative to the level and/or activity of target gene or its product in the absence of modulatory treatment. Particularly because WIP1 shares many features with well known oncogenes as discussed *supra,* inhibition of the WIP1 gene, its protein, or its activities will control or ameliorate precancerous or cancerous conditions, for example, breast cancer, lung cancer, prostate cancer, ovarian cancer, or colon cancer.

The techniques to inhibit or suppress a target gene, for example, WIP1 that is involved in cancers, *i.e.,* the negative modulatory techniques are provided in the present invention. For example, compounds that exhibit negative modulatory activity on WIP 1 can be used in accordance with the invention to prevent and/or ameliorate symptoms of tumors and cancers (for example, breast cancer, lung cancer, prostate cancer, ovarian cancer, or colon cancer). Such molecules can include, but are not limited to, peptides, phosphopeptides, small molecules (molecular weight below about 500), large molecules (molecular weight above about 500), or antibodies (including, for example, polyclonal, monoclonal, humanized, anti-idiotypic, chimeric or single chain antibodies, and Fab, F(ab')₂ and Fab expression library fragments, and epitope-binding fragments thereof), and nucleic acid molecules that interfere with replication, transcription, or translation of the WIP1 gene (for example, antisense nucleic acid molecules, siRNAs and ribozymes).

Antisense, siRNAs and ribozyme molecules that inhibit expression of a target gene, for example, WIP1 may reduce the level of the functional activities of the target gene and its product, for example, reduce the catalytic potency of WIP 1 respectively. Triple helix forming molecules, also related, can be used in reducing the level of target gene activity. These molecules can be designed to reduce or inhibit either wild type, or if appropriate, mutant target gene activity.

For example, anti-sense RNA and DNA molecules act to directly block the translation of mRNA by hybridizing to targeted mRNA and preventing protein translation. With respect to antisense DNA, oligodeoxyribonucleotides derived from the translation initiation site, for example, between the -10 and +10 regions of the target gene nucleotide sequence of interest, are preferred.

Ribozymes are enzymatic RNA molecules capable of catalyzing the specific cleavage of RNA. A review is provided in Rossi, Current Biology, 4:469-471 (1994). The mechanism of ribozyme action involves sequence-specific hybridization of the ribozyme molecule to complementary target RNA, followed by an endonucleolytic cleavage. A composition of ribozyme molecules must include one or more sequences complementary to the target gene mRNA, and must include a well-known catalytic sequence responsible for mRNA cleavage (U.S. Pat. No. 5,093,246). Engineered hammerhead motif ribozyme molecules that may specifically and efficiently catalyze internal cleavage of RNA sequences encoding target protein, for example, WIP1 may be used according to this invention in cancer intervention.

Specific ribozyme cleavage sites within any potential RNA target are initially identified by scanning the molecule of interest, for example, WIP1 RNA, for ribozyme cleavage sites which include the following sequences, GUA, GUU and GUC. Once identified, short RNA sequences of between 15 and 20 ribonucleotides corresponding to the region of the target gene, for example, WIP 1 containing the cleavage site can be evaluated for predicted structural features, for example, secondary structure, that can render an oligonucleotide sequence unsuitable. The suitability of candidate sequences can also be evaluated by testing their accessibility to hybridization with complementary oligonucleotides, using ribonuclease protection assays.

The WIP1 gene sequences also can be employed in an RNA interference context. The phenomenon of RNA interference is described and discussed in Bass, Nature 411: 428-29 (2001); Elbahir et al., Nature 411: 494-98 (2001); and Fire et al., Nature 391: 806-11 (1998), where methods of making interfering RNA also are discussed. The double-stranded RNA based upon the sequence disclosed herein (for example, GenBank accession number NM_025195 for WIP1) is less than 100 base pairs ("bps") in length and constituency and preferably is about 30 bps or shorter, and can be made by approaches known in the art, including the use of complementary DNA strands or synthetic approaches. The RNAs that are capable of causing interference can be referred to as small interfering RNAs ("siRNA"), and can cause post-transcriptional silencing of specific genes in cells, for example, mammalian cells (including human cells) and in the body, for example, mammalian bodies (including humans). Exemplary siRNAs according to the invention could have up to 29 bps, 25 bps, 22 bps, 21 bps, 20 bps, 15 bps, 10 bps, 5 bps or any number thereabout or therebetween.

Nucleic acid molecules that can associate together in a triple-stranded conformation (triple helix) and that thereby can be used to inhibit transcription of a target gene, should be single helices composed of deoxynucleotides. The base composition of these oligonucleotides must be designed to promote triple helix formation via Hoogsteen base pairing rules, which generally require sizeable stretches of either purines or pyrimidines on one strand of a duplex. Nucleotide sequences can be pyrimidine-based, which will result in TAT and CGC triplets across the three associated strands of the resulting triple helix. The pyrimidine-rich molecules provide bases complementary to a purine-rich region of a single strand of the duplex in a parallel orientation to that strand. In addition, nucleic acid molecules can be chosen that are purine-rich, for example, contain a stretch of G residues. These molecules will form a triple helix with a DNA duplex that is rich in GC pairs, in which the majority of the purine residues are located on a single strand of the targeted duplex, resulting in GGC triplets across the three strands in the triplex. Alternatively, the potential sequences that can be targeted for triple helix formation can be increased by creating a so-called "switchback" nucleic acid molecule. Switchback molecules are synthesized in an alternating 5'-3', 3'-5' manner, such that they base pair with first one strand of a duplex and then the other, eliminating the necessity for a sizeable stretch of either purines or pyrimidines on one strand of a duplex.

In instances wherein the antisense, ribozyme, siRNA, and triple helix molecules described herein are used to reduce or inhibit mutant gene expression, it is possible that they can also effectively reduce or inhibit the transcription (for example, using a triple helix) and/or translation (for example, using antisense, ribozyme molecules) of mRNA produced by the normal target gene allele. These situations are pertinent to tumor suppressor genes whose normal levels in the cell or tissue need to be maintained while a mutant is being inhibited. To do this, nucleic acid molecules which are resistant to inhibition by any antisense, ribozyme or triple helix molecules used, and which encode and express target gene polypeptides that exhibit normal target gene activity, can be introduced into cells via gene therapy methods. Alternatively, when the target gene encodes an extracellular protein, it may be preferable to co-administer normal target gene protein into the cell or tissue to maintain the requisite level of cellular or tissue target gene activity. By contrast, in the case of oncogene-like target genes, for example, WIP1, it is the respective normal wild type WIP1 gene and its protein that need to be suppressed. Thus, any mutant or variants that are defective in WIP1 function or that interferes or completely abolishes its normal function would be desirable for cancer treatment. Therefore, the same methodologies described above to safeguard normal gene alleles may be used in the present invention to safeguard the mutants of the target gene in the application of antisense, ribozyme, and triple helix treatment.

Anti-sense RNA and DNA, ribozyme, and triple helix molecules of the invention can be prepared by standard methods known in the art for the synthesis of DNA and RNA molecules. These include techniques for chemically synthesizing oligodeoxyribonucleotides and oligoribonucleotides well known in the art, for example, for example, solid phase phosphoramidite chemical synthesis. Alternatively, RNA molecules can be generated by *in vitro* and *in vivo* transcription of DNA sequences encoding the antisense RNA molecule. Such DNA sequences can be incorporated into a wide variety of vectors which also include suitable RNA polymerase promoters, for example, the T7 or SP6 polymerase promoters. Alternatively, antisense cDNA constructs that synthesize antisense RNA constitutively or inducibly, depending on the promoter used, can be introduced stably into cell lines. Various well-known modifications to the DNA molecules can be introduced as a means for increasing intracellular stability and half-life. Possible modifications include, but are not limited to, the addition of flanking sequences of ribo- or deoxy- nucleotides to the 5' and/or 3' ends of the molecule, or the use of phosphorothioate or 2' O-methyl rather than phosphodiesterase linkages within the oligodeoxyribonucleotide backbone.

In this aspect, the present invention also provides negative modulatory techniques using antibodies. Antibodies can be generated which are both specific for a target gene product and which reduce target gene product activity; they can be administered when negative modulatory techniques are appropriate for the treatment of tumors and cancers, for example, in the case of WIP1 antibodies for breast cancer treatment.

In instances where the target gene protein to which the antibody is directed is intracellular, and whole antibodies are used, internalizing antibodies are preferred. However, lipofectin or liposomes can be used to deliver the antibody, or a fragment of the Fab region which binds to the target gene epitope, into cells. Where fragments of an antibody are used, the smallest inhibitory fragment which specifically binds to the binding domain of the protein is preferred. For example, peptides having an amino acid sequence corresponding to the domain of the variable region of the antibody that specifically binds to the target gene protein can be used. Such peptides can be synthesized chemically or produced by recombinant DNA technology using methods well known in the art (for example, see Creighton, 1983, *supra;* and Sambrook *et al.,* 1989, *supra*). Alternatively, single chain neutralizing antibodies that bind to intracellular target gene product epitopes also can be administered. Such single chain antibodies can be administered, for example, by expressing nucleotide sequences encoding single-chain antibodies within the target cell population by using, for example, techniques, for example, those described in Marasco et al., Proc. Natl. Acad. Sci. U.S.A., 90:7889-7893 (1993). When the target gene protein is extracellular, or is a transmembrane protein, any of the administration techniques known in the art which are appropriate for peptide administration can be used to effectively administer inhibitory target gene antibodies to their site of action. The methods of administration and pharmaceutical preparations are discussed below.

### G. Pharmaceutical Applications of Compounds:

The identified compounds that inhibit the expression, synthesis, and/or activity of the target gene, for example, WIP1 can be administered to a patient at therapeutically effective doses to prevent, treat, or control a tumor or cancer. A therapeutically effective dose refers to an amount of the compound that is sufficient to result in a measurable reduction or elimination of cancer or its symptoms.

Toxicity and therapeutic efficacy of such compounds can be determined by standard pharmaceutical procedures in cell cultures or experimental animals, for example, for determining the LD₅₀ (the dose lethal to 50% of the population) and the ED₅₀ (the dose therapeutically effective in 50% of the population). The dose ratio between toxic and therapeutic effects is the therapeutic index and can be expressed as the ratio, LD₅₀ /ED₅₀. Compounds that exhibit large therapeutic indices are preferred. While compounds that exhibit toxic side effects can be used, care should be taken to design a delivery system that targets such compounds to the site of affected tissue to minimize potential damage to normal cells and, thereby, reduce side effects.

The data obtained from the cell culture assays and animal studies can be used to formulate a dosage range for use in humans. The dosage of such compounds lies preferably within a range of circulating concentrations that include the ED₅₀ with little or no toxicity. The dosage can vary within this range depending upon the dosage form employed and the route of administration. For any compound used in the method of the invention, the therapeutically effective dose can be estimated initially from cell culture assays. A dose can be formulated in animal models to achieve a circulating plasma concentration range that includes the IC₅₀ (the concentration of the test compound that achieves a half-maximal inhibition of symptoms) as determined in cell culture. Such information can be used to more accurately determine useful doses in humans. Levels in plasma can be measured, for example, by high performance liquid chromatography (HPLC).

Pharmaceutical compositions for use in the present invention can be formulated by standard techniques using one or more physiologically acceptable carriers or excipients. The compounds and their physiologically acceptable salts and solvates can be formulated and administered orally, intraorally, rectally, parenterally, epicutaneously, topically, transdermally, subcutaneously, intramuscularly, intranasally, sublingually, intradurally, intraocularly, intrarespiratorally, intravenously, intraperitoneally, intrathecal, mucosally, by oral inhalation, nasal inhalation, or rectal administration, for example.

For oral administration, the pharmaceutical compositions can take the form of tablets or capsules prepared by conventional means with pharmaceutically acceptable excipients, for example, binding agents, for example, pregelatinised maize starch, polyvinylpyrrolidone, or hydroxypropyl methylcellulose; fillers, for example, lactose, microcrystalline cellulose, or calcium hydrogen phosphate; lubricants, for example, magnesium stearate, talc, or silica; disintegrants, for example, potato starch or sodium starch glycolate; or wetting agents, for example, sodium lauryl sulphate. The tablets can be coated by methods well known in the art. Liquid preparations for oral administration can take the form of solutions, syrups, or suspensions, or they can be presented as a dry product for constitution with water or other suitable vehicle before use. Such liquid preparations can be prepared by conventional means with pharmaceutically acceptable additives, for example, suspending agents, for example, sorbitol syrup, cellulose derivatives, or hydrogenated edible fats; emulsifying agents, for example, lecithin or acacia; non-aqueous vehicles, for example, almond oil, oily esters, ethyl alcohol, or fractionated vegetable oils; and preservatives, for example, methyl or propyl-p-hydroxybenzoates or sorbic acid. The preparations can also contain buffer salts, flavoring, coloring, and/or sweetening agents as appropriate. Preparations for oral administration can be suitably formulated to give controlled release of the active compound.

For administration by inhalation, the compounds are conveniently delivered in the form of an aerosol spray presentation from pressurized packs or a nebulizer, with the use of a suitable propellant, for example, dichlorodifluoromethane, trichlorofluoromethane, dichlorotetrafluoroethane, carbon dioxide, or other suitable gas. In the case of a pressurized aerosol, the dosage unit can be determined by providing a valve to deliver a metered amount. Capsules and cartridges of, for example, gelatin for use in an inhaler or insufflator can be formulated containing a powder mix of the compound and a suitable powder base, for example, lactose or starch.

The compounds can be formulated for parenteral administration by injection, for example, by bolus injection or continuous infusion. Formulations for injection can be presented in unit dosage form, for example, in ampoules or in multi-dose containers, with an added preservative. The compositions can take such forms as suspensions, solutions, or emulsions in oily or aqueous vehicles, and can contain formulatory agents, for example, suspending, stabilizing, and/or dispersing agents. Alternatively, the active ingredient can be in powder form for constitution with a suitable vehicle, for example, sterile pyrogen-free water, before use. The compounds can also be formulated in rectal compositions, for example, suppositories or retention enemas, for example, containing conventional suppository bases, for example, cocoa butter or other glycerides.

Furthermore, the compounds can also be formulated as a depot preparation. Such long acting formulations can be administered by implantation (for example, subcutaneously or intramuscularly) or by intramuscular injection. Thus, for example, the compounds can be formulated with suitable polymeric or hydrophobic materials (for example as an emulsion in an acceptable oil) or ion exchange resins, or as sparingly soluble derivatives, for example, as a sparingly soluble salt.

The compositions can, if desired, be presented in a pack or dispenser device which can contain one or more unit dosage forms containing the active ingredient. The pack can for example comprise metal or plastic foil, for example, a blister pack. The pack or dispenser device can be accompanied by instructions for administration.

The invention is .further described by the following examples, which do not limit the invention in any manner.

### EXAMPLES:

### Example I: Amplification of the WIP1 Gene:

The present inventors used DNA microarray-based CGH to survey the genome for gene amplification, and discovered that the WIP1 gene is frequently amplified in tumor tissue and cell lines.

The genomic DNAs were isolated from breast cancer, lung cancer, prostate cancer, ovarian cancer, and colon cancer cell lines. They were subjected, along with the same WIP 1 TaqMan probe set as described *supra* representing the target, and a reference probe representing a normal non-amplified, single copy region in the genome, to analysis by TaqMan 7700 Sequence Detector following the manufacturer's protocol. Table 1 shows the number of the WIP 1 genes in each sample from cancer cell lines. Out of 11 breast cancer cell lines tested, BT474, MDAMB361, ZR75-30, MCF7, ZR75-1, MDAMB134, MDAMB453, MDAMB 157, MDAMB 175, MDAMB330, and MDAMB231, four of them BT474, MDAMB361, ZR75-30, and MCF7 were observed to have at least a 2.5 folds increase in their WIP1 DNA copies, which gives rise to an amplification frequency of 4/11, i.e., over 36%.

Only samples with the WIP1 gene copy number greater than or equal to 2.5 fold are deemed to have been amplified, because of the instrumental detection limit. That is, for example, a Taqman 7700 instrument can not easily distinguish one copy from a two-fold increase in gene copies. However, an increase in WIP1 gene copy number less than 2.5 fold can still be considered as an amplification of the gene.

TaqMan epicenter data for WIP1: Referring to Figure 2, the indicated cell lines or primary tumors were examined for DNA copy number of genes and markers near WIP1 to map the boundaries of the amplified regions. WIP1 was found at the epicenter.

### Example II: Overexpression of the WIP1 Gene in Cancer Cell Lines:

Reverse transcriptase (RT)-directed quantitative PCR was performed using the TaqMan 7700 Sequence Detector (Applied Biosystems) to determine the WIP1 mRNA level in each sample. Human beta-actin mRNA was used as control. The nucleotide sequences of the WIP1 were used to design and make a suitable TaqMan probe set for WIP1 (see GENBANK RECORD AAB61637). The measurements of the mRNA level of each cancer cell line sample were normalized to the mRNA levels in normal mammary epithelial cell samples. Of 11 breast cancer cell lines tested (BT474, MDAMB361, ZR75-30, MCF7, ZR75-1, MDAMB134, MDAMB453, MDAMB 157, MDAMB 175, MDAMB330, MDAMB231) exhibit WIP 1 overexpression of over 4.5 folds (See Table 2).

### Example III: Physical Map of the Amplicon Containing the WIP1 Gene Locus:

The present inventors further demonstrated that WIP 1 is located at the epicenter of the amplification regions (Figure 2). Figure 2 shows the epicenter mapping of 17q23 amplicon which includes WIP 1 locus. The number of DNA copies for each sample is plotted on the Y-axis, and the X-axis corresponds to nucleotide position based on Human Genome Project working draft sequence (http://genome.ucsc.edu/goldenPath/aug2001Tracks.html). The DNA copy numbers were evaluated using Q-PCR and fluorogenic Taqman probes were designed based on ESTs or BAC sequences. The markers were ordered on the basis of their physical presence on the BACs. High-resolution DNA copy number profiles for breast cancer cell lines MCF7 and ZR75-30 and primary invasive breast tumors 87-637 and 87-320 are depicted for a 1.3-Mb region surrounding the PAT1 and WIP1 genes. Included are all known genes or spliced cDNAs telomeric to USP (AF350251), including PAT1, WIP1, and the 5' end of FLJ21857; however, for clarity on the centromeric side, only the gene encoding a subunit of S6-kinase is shown. To determine the DNA copy number for each of the gene, corresponding probes to each marker were designed using PrimerExpress 1.0 (Applied Biosystems) and synthesized by Operon Technologies. Subsequently, the target probe (representing the marker), a reference probe (representing a normal non-amplified, single copy region in the genome), and tumor genomic DNA (10 ng) were subjected to analysis by the Applied Biosystems 7700 TaqMan Sequence Detector following the manufacturer's protocol. Example of amplification is shown in Figure 2. Only one full-length gene WIP1 was in this epicenter, along with the gene PAT1. The overall amplification and overexpression frequencies of WIP 1 are shown in Tables 1 and 2.

### Example IV: Detection of Endogenous WIP1 Protein

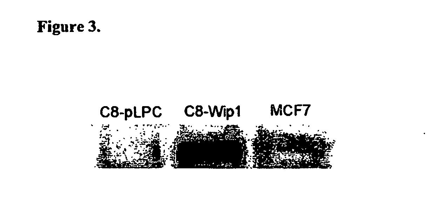

Rabbit polyclonal antibody was used to detect WIP1 protein in C8 retrovirus infected stable cell lines, and the endogenous WIP1 from breast cancer cell line MCF7. WIP1 protein levels in mouse embryonic fibroblast C8 cells stably transfected with vector alone (pLPC) or WIP1, and breast cancer cell line MCF7 were measured by Western blot (see Figure 3). Rabbit polyclonal antibody was generated from recombinant WIP1.

### Example V: Assays for Oncogenic Function of PAT1 and WIP1 Genes

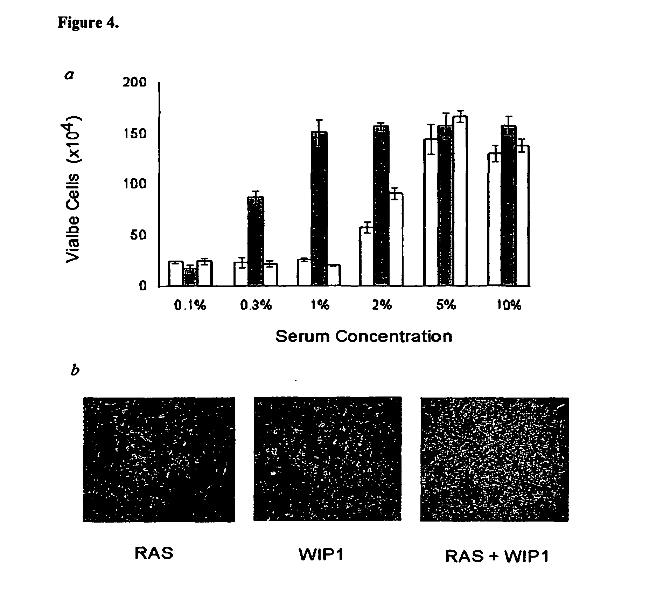

Figure 4 shows results of the assays for oncogenic function of PAT 1 and WIP1 genes. The number of viable cells after 48 hours of incubation in the presence of the indicated serum concentration is depicted (See Figure 4a). The empty pLPC vector (white bars), pLPC-WIP1 (dark gray bars), and pLPC-PAT1 (stippled bars) were introduced by retroviral transfection into primary mouse embryo fibroblasts transformed with E1A and RAS. These cells undergo apoptosis when starved for serum. Following selection in puromycin, 1 x 10⁶ transfected cells were plated in triplicate onto 35 mm plates. After a 16-hour incubation in serum-free medium, the cells were harvested and then cultured for 48 hours in Delbecco's Modified Eagle Medium (DMEM) with the indicated concentration of fetal bovine serum. The number of viable cells were determined using trypan blue exclusion and a hemacytometer. A typical transformed foci of mouse embryo fibroblasts that had been infected with retroviral constructs containing WIP1 and mutationally activated RAS is depicted along with representative areas of surviving cells following infection with either the RAS or WIP1 vectors alone (See Figure 4b). Semi-confluent 100-mm dishes of primary mouse embryo fibroblasts were transfected with pLPC-derived vectors, split 1:3, and selected with puromycin for 4 days. After an additional 3 weeks of incubation, all colonies and areas of growth in plates containing cells infected with either the WIP1 or RAS vectors had significantly receded, wheras WIP1/RAS co-transfectants formed 5 to 10 highly transformed foci. These findings were observed in two separate experiments. Four such foci were cloned and determined to overexpress WIP1. WIP1 overexpression significantly attenuated apoptosis induced by serum-starvation (See Figure 4a). WIP1 cooperated with mutationally activated RAS to transform primary mouse fibroblasts (See Figure 4b).

### Example VI: WIP1 Suppresses Apoptosis Induced by TNF-α

WIP 1 contributes to suppression of the UV-induced apoptosis by negatively feedback on the p38-p53 signaling. The present inventors demonstrated that expression of WIP1 protects TNF-α induced apoptosis in a p53 independent manner. Referring to Figure 5, apoptosis experiments were performed using mouse embryonic fibroblasts that were immortalized with oncogenes E1A and RAS. C8 cells which is derived from p53+/+ mouse embryonic fibroblast that is immortalized with E1A and RAS oncogenes, were stably infected with retrovirus containing just vector alone (pLPC) or WIP1 (pLPC-WIP1) or PAT I (pLPC-PAT1). Cell line A9 was derived from p53-/- mouse embryonic fibroblasts and A9 cells were stably infected with retrovirus containing just vector alone (pLPC) representing the control, WIP I (pLPC-WIP1) representing the test, or Bcl2 (pLPC-BcL2) representing a comparison. For TNF-α induced apoptosis, the medium were supplemented with 10 or 20 ng/ml TNF-α, the number of viable cells were determined using trypan blue exclusion and a hemacytometer. For UV induced apoptosis, the cells were treated with 25 J/m2, 50 J/m2, or 75 J/m2 UV radiation, the cell death was assessed by counting viable cells using trypan blue exclusion and a hemacytometer. Two kinds of stimuli were employed to induce apoptosis, i.e., serum starvation and cytokine TNF-α. For serum starvation assay, the cells were grown in 0.1% FBS for 48 hours; then, the apoptotic phenotype was visualized and assessed.

Columns 4, 5, and 6 of the Table 3 show the result of apoptosis assessment under different conditions. The term "resistance" indicates that the majority of the cells were healthy. The term "apoptosis" indicates that extensive apoptotic-like cell death were observed. Unlike BcL2, which protects cells from serum starvation induced apoptosis (see Table 3 column 6, rows 1, 2), WIP1 has no effect on serum starvation induced apoptosis in the presence of p53 (see Table 3 column 5, row 1). However, WIP1 protects cells from cytokine TNF-α induced apoptosis with no regard to the presence of p53 (see Table 3 column 5, rows 3, 4). Such effect of WIP1 thus further implicates its role in cancer formation and development, as illustrated in Figure 1 (step 106).

**Table 3. WIP1 Suppresses Apoptosis Induced by TNF-α**

| **CELL LINE** | **p53** | **CONDITION** | **pLPC** | **pLPC-WIP1** | **pLPC-BeL2** |
|---|---|---|---|---|---|
| C8 | + | serum starvation | Apoptosis | Apoptosis | Resistance |
| A9 | - | serum starvation | Resistance | Resistance | Resistance |
| C8 | + | TNF-α (20 ng ml⁻¹) | Apoptosis | Resistance | Apoptosis |
| A9 | - | TNF-α (20 ng ml⁻¹) | Apoptosis | Resistance | Apoptosis |

**SEQ ID NO:1. Human WIP1 DNA sequence:** The GenBank accession number for WIP1 is NM_003620.

**SEQ ID NO:2. Human WIP1 Polypeptide sequence:** The GenBank accession number is NM_003620.

## Claims

1. A method for diagnosing a cancer in a mammal, comprising:
determining the WIP1 gene copy number in a test sample from a region of the mammal that is suspected to be precancerous or cancerous, thereby generating data for a test gene copy number; and
comparing the test gene copy number to data for a control gene copy number, wherein an amplification of the gene in the test sample relative to the control indicates the presence of a precancerous lesion or a cancer in the mammal.

2. Use of a nucleotide molecule which interacts with WIP1 DNA or RNA to inhibit WIP1 gene function in the manufacture of a medicament for the inhibition of cancer or precancerous growth in a mammalian tissue, said nucleotide molecule selected from an siRNA, an antisense nucleic acid molecule, and a ribozyme of WIP1 RNA.

3. Use according to claim 2, wherein the siRNA comprises less than about 100 bps in length.

4. Use of an antibody to WIP1 protein in the manufacture of a medicament for inhibiting cancer or precancerous growth in a mammalian tissue.

5. A method for diagnosing a cancer in a mammal, comprising:
determining the level of WIP1 protein expression or WIP1 mRNA transcripts in a test sample from a region of the mammal that is suspected to be precancerous or cancerous, thereby generating data for a test level; and
comparing the test level to data for a control level, wherein an elevated test level of the test sample relative to the control level indicates the presence of a precancerous lesion or cancer in the mammal.

6. A method according to claim 5, wherein the data for a control level is obtained from an electronic or paper format, wherein the electronic format is selected from electronic mail, disk, compact disk (CD), digital versatile disk (DVD), memory card, memory chip, ROM or RAM, magnetic optical disk, tape, video, video clip, microfilm, internet shared network, shared server; wherein the data is displayed, transmitted or analysed via physical transfer, electronic transmission, video display, or telecommunication; wherein the data is compared and compiled at the site of sampling specimens or at a location where the data is transmitted.

7. Use of a siRNA molecule, wherein the siRNA is able to interact with WIP1 gene or WIP1 mRNA transcript, in the manufacture of a medicament for the treatment or inhibition of cancer or precancerous growth in a mammalian tissue.

8. Use according to claim 7, wherein the siRNA molecule is in the form of a vector and wherein the vector is a plasmid, cosmid or bacteriophage.

9. A method for determining the efficacy of a therapeutic treatment regimen in a patient, comprising:
measuring the WIP1 gene copy number in a first sample of suspected precancerous or cancerous cells obtained from a patient, thereby generating an initial level;
measuring the WIP1 gene copy number in a second sample of suspected precancerous or cancerous cells from the patient at a time following the administration of the treatment regimen, thereby generating a test level; and
comparing the initial and the test levels, wherein a decrease in the gene copy number levels in the test level relative to the initial level indicates that the treatment regimen is effective in the patient.

10. A method for determining the efficacy of a therapeutic treatment regimen in a patient, comprising:
a) measuring at least one of WIP1 mRNA or WIP1 protein expression levels in a first sample of suspected precancerous or cancerous cells obtained from a patient, thereby generating data for a pre-treatment level;
b) measuring at least one of WIP1 mRNA or WIP1 protein expression levels in a second sample of suspected precancerous or cancerous cells from the patient at a time following administration of the treatment regimen, thereby generating data for a test level; and
c) comparing the pre-treatment level with the test level, wherein data showing no decrease in the test level relative to the pre-treatment level indicates that the treatment regimen is not effective in the patient.

11. The method or use according to any preceding claim, wherein the sample or tissue is selected from breast tissue, lung tissue, prostate tissue, ovarian tissue and colon tissue.

12. An isolated WIP1 gene amplicon, wherein the amplicon comprises more than one copy of a polynucleotide selected from:
(a) a polynucleotide encoding the polypeptide set forth in SEQ ID NO: 2;
(b) a polynucleotide set forth in SEQ ID NO:1;
(c) a polynucleotide having at least about 90% sequence identity to the polynucleotide of (a) or (b); and
(d) a polynucleotide that is overexpressed in tumor cells having at least about 90% sequence identity to the polynucleotide of (a) or (b).

## Patentansprüche

1. Verfahren zum Diagnostizieren von Krebs in einem Säugetier, das die folgenden Schritte beinhaltet:
Bestimmen der WIP1-Genkopiezahl in einer Testprobe aus einer Region des Säugetieres, die vermutlich präkanzerös oder kanzerös ist, wodurch Daten für eine Test-Genkopiezahl erzeugt werden; und
Vergleichen der Test-Genkopiezahl mit Daten für eine Kontroll-Genkopiezahl, wobei eine Amplifikation des Gens in der Testprobe relativ zur Kontrolle die Anwesenheit einer präkanzerösen Läsion oder von Krebs in dem Säugetier anzeigt.

2. Verwendung eines Nucleotidmoleküls, das mit WIP1 DNA oder RNA interagiert, um die WIP1-Genfunktion zu inhibieren, bei der Herstellung eines Medikamentes zur Inhibition von Krebs oder präkanzerösem Wachstum in Säugetiergewebe, wobei das genannte Nucleotidmolekül ausgewählt ist aus einer siRNA, einem Antisense-Nucleinsäuremolekül und einem Ribozym von WIP1 RNA.

3. Verwendung nach Anspruch 2, wobei die siRNA eine Länge von weniger als etwa 100 bp hat.

4. Verwendung eines Antikörpers gegen WIP1-Protein bei der Herstellung eines Medikamentes zur Inhibition von Krebs oder präkanzerösem Wachstum in Säugetiergewebe.

5. Verfahren zum Diagnostizieren von Krebs in einem Säugetier, das die folgenden Schritte beinhaltet:
Bestimmen des Niveaus von WIP1-Proteinexpression oder WIP1 mRNA-Transkipten in einer Testprobe von einer Region des Säugetiers, die vermutlich präkanzerös oder kanzerös ist, wodurch Daten für ein Testniveau erzeugt werden; und
Vergleichen des Testniveaus mit Daten für ein Kontrollniveau, wobei ein erhöhtes Testniveau der Testprobe relativ zum Kontrollniveau auf die Anwesenheit einer präkanzerösen Läsion oder Krebs in dem Säugetier hinweist.

6. Verfahren nach Anspruch 5, wobei die Daten für ein Kontrollniveau von einem Elektronik- oder Papierformat erhalten werden, wobei das Elektronikformat ausgewählt wird aus elektronischer Post, Diskette, Compact Disk (CD), Digital Versatile Disk (DVD), Speicherkarte, Speicherchip, ROM oder RAM, MO-Diskette, Band, Video, Videoclip, Mikrofilm, per Internet gemeinsam genutztem Netzwerk, Shared Server; wobei die Daten angezeigt, übertragen oder analysiert werden per physikalischem Transfer, elektronischer Übertragung, Videoanzeige oder Telekommunikation; wobei die Daten an dem Ort der Probenahme oder an einem Ort verglichen und zusammengetragen werden, zu dem die Daten übertragen werden.

7. Verwendung eines siRNA-Moleküls, wobei die siRNA mit dem WIP1-Gen oder WIP1 mRNA-Transkript interagieren kann, bei der Herstellung eines Medikaments zur Behandlung oder Inhibition von Krebs oder präkanzerösem Wachstum in Säugetiergewebe.

8. Verwendung nach Anspruch 7, wobei das siRNA-Molekül in Form eines Vektors vorliegt und wobei der Vektor ein Plasmid, Cosmid oder Bakteriophage ist.

9. Verfahren zum Bestimmen der Wirksamkeit eines therapeutischen Behandlungsplans bei einem Patienten, das die folgenden Schritte beinhaltet:
Messen der WIP1-Genkopiezahl in einer ersten Probe mutmaßlicher präkanzeröser oder kanzeröser Zellen, die von einem Patienten erhalten wurde, wodurch ein Ausgangsniveau erzeugt wird;
Messen der WIP1-Genkopiezahl in einer zweiten Probe mutmaßlicher präkanzeröser oder kanzeröser Zellen von dem Patienten zu einem Zeitpunkt nach der Anwendung des Behandlungsplans, wodurch ein Testniveau erzeugt wird; und
Vergleichen des Ausgangsniveaus mit dem Testniveau, wobei eine Abnahme des Genkopiezahlniveaus im Testniveau relativ zum Ausgangsniveau anzeigt, dass der Behandlungsplan für den Patienten wirksam ist.

10. Verfahren zum Bestimmen der Wirksamkeit eines therapeutischen Behandlungsplans bei einem Patienten, das die folgenden Schritte beinhaltet:
a) Messen des WIP1 mRNA und/oder WIP1-Protein-Expressionsniveaus an einer ersten Probe mutmaßlicher präkanzeröser oder kanzeröser Zellen, die von einem Patienten erhalten wurde, wodurch Daten für ein Vorbehandlungsniveau erzeugt werden;
b) Messen des WIP1 mRNA und/oder WIP1-Protein-Expressionsniveaus an einer zweiten Probe mutmaßlicher präkanzeröser oder kanzeröser Zellen von dem Patienten zu einem Zeitpunkt nach der Anwendung des Behandlungsplans, wodurch Daten für ein Testniveau erzeugt werden; und
c) Vergleichen des Vorbehandlungsniveaus mit dem Testniveau, wobei Daten, die keine Abnahme des Testniveaus relativ zum Vorbehandlungsniveau aufweisen, zeigen, dass der Behandlungsplan für den Patienten nicht wirksam ist.

11. Verfahren oder Verwendung nach einem der vorangehenden Ansprüche, wobei die Probe oder das Gewebe ausgewählt wird aus Brustgewebe, Lungengewebe, Prostatagewebe, Eierstockgewebe und Kolongewebe.

12. Isoliertes WIP1-Genamplicon, wobei das Amplicon mehr als eine Kopie eines Polynucleotids umfasst, ausgewählt aus:
(a) einem Polynucleotid, das das in SEQ ID Nr. 2 dargelegte Polypeptid kodiert;
(b) einem Polynucleotid, das in SEQ ID Nr. 1 dargelegt ist;
(c) einem Polynucleotid mit wenigstens etwa 90 % Sequenzidentität mit dem Polynucleotid aus (a) oder (b); und
(d) einem Polynucleotid, das in Tumorzellen überexprimiert ist, mit wenigstens etwa 90 % Sequenzidentität mit dem Polynucleotid aus (a) oder (b).

## Revendications

1. Méthode de diagnostic d'un cancer chez un mammifère, comprenant:
déterminer le nombre de copies du gène WIP 1 dans un échantillon de test provenant d'une région du mammifère qui est soupçonnée être précancéreuse ou cancéreuse, produisant ainsi des données destinées à un nombre de test de copies du gène; et
comparer le nombre de test de copies du gène à des données destinées à un nombre témoin de copies du gène, où une amplification du gène dans l'échantillon de test par rapport au témoin, indique la présence d'une lésion précancéreuse ou d'un cancer chez le mammifère.

2. Utilisation d'une molécule de nucléotides qui interagit avec l'ADN ou l'ARN de WIP 1 pour inhiber la fonction du gène WIP 1, dans la fabrication d'un médicament pour l'inhibition du cancer ou de croissance précancéreuse dans un tissu mammalien, ladite molécule de nucléotides sélectionnée d'un siRNA, d'une molécule d'acide nucléique anti-sens et d'un ribozyme d'ARN de WIP1.

3. Utilisation selon la revendication 2, dans laquelle le siRNA comprend moins qu'environ 100 pb de longueur.

4. Utilisation d'un anticorps à la protéine WIP1, dans la fabrication d'un médicament pour inhiber un cancer ou une croissance précancéreuse dans un tissu mammalien.

5. Méthode de diagnostic d'un cancer chez un mammifère, comprenant:
déterminer le niveau d'expression de la protéine WIP1 ou de produits de transcription d'ARNm de WIP1 dans un échantillon de test provenant d'une région du mammifère qui est soupçonnée être précancéreuse ou cancéreuse, produisant ainsi des données destinées à un niveau de test; et
comparer le niveau de test à des données destinées à un niveau témoin, où un niveau de test élevé de l'échantillon de test par rapport au niveau témoin, indique la présence d'une lésion précancéreuse ou d'un cancer chez le mammifère.

6. Méthode selon la revendication 5, dans laquelle les données destinées à un niveau témoin sont obtenues d'un format électronique ou sur papier, où le format électronique est sélectionné parmi courrier électronique, disquette, disque compact (CD), disque vidéo numérique (DVD), carte mémoire, puce mémoire, ROM ou RAM, disque optique magnétique, bande, vidéo, clip vidéo, microfilm, réseau Internet partagé, serveur partagé; dans laquelle les données sont affichées, transmises ou analysées par transfert physique, transmission électronique, affichage vidéo ou télécommunication; dans laquelle les données sont comparées et compilées au site d'échantillonnage des spécimens ou à un emplacement où les données sont transmises.

7. Utilisation d'une molécule de siRNA, où le siRNA est capable d'interagir avec le gène WIP1 ou un produit de transcription d'ARNm de WIP1, dans la fabrication d'un médicament pour le traitement ou l'inhibition d'un cancer ou d'une croissance précancéreuse dans un tissu mammalien.

8. Utilisation selon la revendication 7, où la molécule de siRNA est sous forme de vecteur et où le vecteur est un plasmide, un cosmide ou un bactériophage.

9. Méthode de détermination de l'efficacité d'un schéma de traitement thérapeutique chez un patient, comprenant:
mesurer le nombre de copies du gène WIP1 dans un premier échantillon de cellules soupçonnées être précancéreuses ou cancéreuses obtenu d'un patient, produisant ainsi un niveau initial;
mesurer le nombre de copies du gène WIP1 dans un deuxième échantillon de cellules soupçonnées être précancéreuses ou cancéreuses d'un patient à un moment suite à l'administration du schéma de traitement, produisant ainsi un niveau de test; et
comparer le niveau initial et le niveau de test, où une réduction dans les niveaux du nombre de copies du gène dans le niveau de test par rapport au niveau initial, indique que le schéma de traitement est efficace chez le patient.

10. Méthode de détermination de l'efficacité d'un schéma de traitement thérapeutique chez un patient, comprenant:
a) mesurer au moins l'un d'entre le niveau d'expression d'ARNm de WIP1 ou le niveau d'expression de la protéine WIP1 dans un premier échantillon de cellules soupçonnées être précancéreuses ou cancéreuses obtenu d'un patient, produisant ainsi des données destinées à un niveau de pré-traitement;
b) mesurer au moins l'un d'entre le niveau d'expression d'ARNm de WIP1 ou le niveau d'expression de la protéine WIP1 dans un deuxième échantillon de cellules soupçonnées être précancéreuses ou cancéreuses du patient à un moment suite à l'administration du schéma de traitement, produisant ainsi des données destinées à un niveau de test; et
c) comparer le niveau de pré-traitement au niveau de test, où des données qui ne présentent pas de réduction dans le niveau de test par rapport au niveau de pré-traitement, indiquent que le schéma de traitement n'est pas efficace chez le patient.

11. La méthode ou l'utilisation selon l'une quelconque des revendications précédentes, dans laquelle l'échantillon ou le tissu est sélectionné parmi un tissu du sein, un tissu pulmonaire, un tissu de la prostate, un tissu ovarien ou un tissu du côlon.

12. Amplicon du gène WIP1 isolé, où l'amplicon comprend plus d'une copie d'un polynucléotide sélectionné parmi:
(a) un polynucléotide codant le polypeptide présenté dans la SEQ ID NO:2;
(b) un polynucléotide présenté dans la SEQ ID NO:1;
(c) un polynucléotide ayant au moins environ 90% d'identité de séquence avec le polynucléotide de (a) ou de (b); et
(d) un polynucléotide qui est surexprimé dans des cellules tumorales, ayant au moins environ 90% d'identité de séquence avec le polynucléotide de (a) ou de (b).


### SEQUENCE LISTING

<110> TULARIK INC.
<120> AMPLIFIED CANCER GENE WIP1
<130> 38002-0021
<140> PCT/US02/03991
   <141> 2002-02-12
<150> US 60/268,362
   <151> 2001-02-14
<160> 3
<170> PatentIn version 3.1
<210> 1
<211> 1818
<212> DNA
<213> Homo sapiens
<400> 1
   atggcggggc tgtactcgct gggagtgagc gtcttctccg accagggcgg gaggaagtac     60
   atggaggacg ttactcaaat cgttgtggag cccgaaccga cggctgaaga aaagccctcg    120
   ccgcggcggt cgctgtctca gccgttgcct ccgcggccgt cgccggccgc ccttcccggc    180
   ggcgaagtct cggggaaagg cccagcggtg gcagcccgag aggctcgcga ccctctcccg    240
   gacgccgggg cctcgccggc acctagccgc tgctgccgcc gccgttcctc cgtggccttt    300
   ttcgccgtgt gcgacgggca cggcgggcgg gaggcggcac agtttgcccg ggagcacttg    360
   tggggtttca tcaagaagca gaagggtttc acctcgtccg agccggctaa ggtttgcgct    420
   gccatccgca aaggctttct cgcttgtcac cttgccatgt ggaagaaact ggcggaatgg    480
   ccaaagacta tgacgggtct tcctagcaca tcagggacaa ctgccagtgt ggtcatcatt    540
   cggggcatga agatgtatgt agctcacgta ggtgactcag gggtggttct tggaattcag    600
   gatgacccga aggatgactt tgtcagagct gtggaggtga cacaggacca taagccagaa    660
   cttcccaagg aaagagaacg aatcgaagga cttggtggga gtgtaatgaa caagtctggg    720
   gtgaatcgtg tagtttggaa acgacctcga ctcactcaca atggacctgt tagaaggagc    780
   acagttattg accagattcc ttttctggca gtagcaagag cacttggtga tttgtggagc    840
   tatgatttct tcagtggtga atttgtggtg tcacctgaac cagacacaag tgtccacact    900
   cttgaccctc agaagcacaa gtatattata ttggggagtg atggactttg gaatatgatt    960
   ccaccacaag atgccatctc aatgtgccag gaccaagagg agaaaaaata cctgatgggt   1020
   gagcatggac aatcttgtgc caaaatgctt gtgaatcgag cattgggccg ctggaggcag   1080
   cgtatgctcc gagcagataa cactagtgcc atagtaatct gcatctctcc agaagtggac   1140
   aatcagggaa actttaccaa tgaagatgag ttatacctga acctgactga cagcccttcc   1200
   tataatagtc aagaaacctg tgtgatgact ccttccccat gttctacacc accagtcaag   1260
   tcactggagg aggatccatg gccaagggtg aattctaagg accatatacc tgccctggtt   1320
   cgtagcaatg ccttctcaga gaatttttta gaggtttcag ctgagatagc tcgagagaat   1380
   gtccaaggtg tagtcatacc ctcaaaagat ccagaaccac ttgaagaaaa ttgcgctaaa   1440
   gccctgactt taaggataca tgattctttg aataatagcc ttccaattgg ccttgtgcct   1500
   actaattcaa caaacactgt catggaccaa aaaaatttga agatgtcaac tcctggccaa   1560
   atgaaagccc aagaaattga aagaacccct ccaacaaact ttaaaaggac attagaagag   1620
   tccaattctg gccccctgat gaagaagcat agacgaaatg gcttaagtcg aagtagtggt   1680
   gctcagcctg caagtctccc cacaacctca cagcgaaaga actctgttaa actcaccatg   1740
   cgacgcagac ttaggggcca gaagaaaatt ggaaatcctt tacttcatca acacaggaaa   1800
   actgtttgtg tttgctga                                                 1818
<210> 2
<211> 605
<212> PRT
<213> Homo sapiens
<400> 2
<210> 3
<211> 2973
<212> DNA
<213> Homo sapiens
<400> 3
   ctggctctgc tcgctccggc gctccggccc agctctcgcg gacaagtcca gacatcgcgc     60
   gccccccctt ctccgggtcc gccccctccc ccttctcggc gtcgtcgaag ataaacaata    120
   gttggccggc gagcgcctag tgtgtctccc gccgccggat tcggcgggct gcgtgggacc    180
   ggcgggatcc cggccagccg gccatggcgg ggctgtactc gctgggagtg agcgtcttct    240
   ccgaccaggg cgggaggaag tacatggagg acgttactca aatcgttgtg gagcccgaac    300
   cgacggctga agaaaagccc tcgccgcggc ggtcgctgtc tcagccgttg cctccgcggc    360
   cgtcgccggc cgcccttccc ggcggcgaag tctcggggaa aggcccagcg gtggcagccc    420
   gagaggctcg cgaccctctc ccggacgccg gggcctcgcc ggcacctagc cgctgctgcc    480
   gccgccgttc ctccgtggcc tttttcgccg tgtgcgacgg gcacggcggg cgggaggcgg    540
   cacagtttgc ccgggagcac ttgtggggtt tcatcaagaa gcagaagggt ttcacctcgt    600
   ccgagccggc taaggtttgc gctgccatcc gcaaaggctt tctcgcttgt caccttgcca    660
   tgtggaagaa actggcggaa tggccaaaga ctatgacggg tcttcctagc acatcaggga    720
   caactgccag tgtggtcatc attcggggca tgaagatgta tgtagctcac gtaggtgact    780
   caggggtggt tcttggaatt caggatgacc cgaaggatga ctttgtcaga gctgtggagg    840
   tgacacagga ccataagcca gaacttccca aggaaagaga acgaatcgaa ggacttggtg    900
   ggagtgtaat gaacaagtct ggggtgaatc gtgtagtttg gaaacgacct cgactcactc    960
   acaatggacc tgttagaagg agcacagtta ttgaccagat tccttttctg gcagtagcaa   1020
   gagcacttgg tgatttgtgg agctatgatt tcttcagtgg tgaatttgtg gtgtcacctg   1080
   aaccagacac aagtgtccac actcttgacc ctcagaagca caagtatatt atattgggga   1140
   gtgatggact ttggaatatg attccaccac aagatgccat ctcaatgtgc caggaccaag   1200
   aggagaaaaa atacctgatg ggtgagcatg gacaatcttg tgccaaaatg cttgtgaatc   1260
   gagcattggg ccgctggagg cagcgtatgc tccgagcaga taacactagt gccatagtaa   1320
   tctgcatctc tccagaagtg gacaatcagg gaaactttac caatgaagat gagttatacc   1380
   tgaacctgac tgacagccct tcctataata gtcaagaaac ctgtgtgatg actccttccc   1440
   catgttctac accaccagtc aagtcactgg aggaggatcc atggccaagg gtgaattcta   1500
   aggaccatat acctgccctg gttcgtagca atgccttctc agagaatttt ttagaggttt   1560
   cagctgagat agctcgagag aatgtccaag gtgtagtcat accctcaaaa gatccagaac   1620
   cacttgaaga aaattgcgct aaagccctga ctttaaggat acatgattct ttgaataata   1680
   gccttccaat tggccttgtg cctactaatt caacaaacac tgtcatggac caaaaaaatt   1740
   tgaagatgtc aactcctggc caaatgaaag cccaagaaat tgaaagaacc cctccaacaa   1800
   actttaaaag gacattagaa gagtccaatt ctggccccct gatgaagaag catagacgaa   1860
   atggcttaag tcgaagtagt ggtgctcagc ctgcaagtct ccccacaacc tcacagcgaa   1920
   agaactctgt taaactcacc atgcgacgca gacttagggg ccagaagaaa attggaaatc   1980
   ctttacttca tcaacacagg aaaactgttt gtgtttgctg aaatgcatct gggaaatgag   2040
   gtttttccaa acttaggata taagagggct ttttaaattt ggtgccgatg ttgaactttt   2100
   tttaagggga gaaaattaaa agaaatatac agtttgactt tttggaattc agcagtttta   2160
   tcctggcctt gtacttgctt gtattgtaaa tgtggatttt gtagatgtta gggtataagt   2220
   tgctgtaaaa tttgtgtaaa tttgtatcca cacaaattca gtctctgaat acacagtatt   2280
   cagagtctct gatacacagt aattgtgaca atagggctaa atgtttaaag aaatcaaaag   2340
   aatctattag attttagaaa aacatttaaa ctttttaaaa tacttattaa aaaatttgta   2400
   taagccactt gtcttgaaaa ctgtgcaact ttttaaagta aattattaag cagactggaa   2460
   aagtgatgta ttttcatagt gacctgtgtt tcacttaatg tttcttagag ccaagtgtct   2520
   tttaaacatt attttttatt tctgatttca taattcagaa ctaaattttt catagaagtg   2580
   ttgagccatg ctacagttag tcttgtccca attaaaatac tatgcagtat ctcttacatc   2640
   agtagcattt ttctaaaacc ttagtcatca gatatgctta ctaaatcttc agcatagaag   2700
   gaagtgtgtt tgcctaaaac aatctaaaac aattcccttc tttttcatcc cagaccaatg   2760
   gcattattag gtcttaaagt agttactccc ttctcgtgtt tgcttaaaat atgtgaagtt   2820
   ttccttgcta tttcaataac agatggtgct gctaattccc aacatttctt aaattatttt   2880
   atatcataca gttttcattg attatatggg tatatattca tctaataaat cagtgaactg   2940
   ttcctcatgt tgctgaaaaa aaaaaaaaaa aaa                                 2973